(19) Europäisches Patentamt
European Patent Office
Office européen des brevets

(11) EP 2 198 844 A2

(12) DEMANDE DE BREVET EUROPEEN

(43) Date de publication:
**23.06.2010 Bulletin 2010/25**

(21) Numéro de dépôt: **09179684.7**

(22) Date de dépôt: **17.12.2009**

(51) Int Cl.:
***A61K 8/34** (2006.01)* ***A61K 8/37** (2006.01)*
***A61K 8/72** (2006.01)* ***A61K 8/73** (2006.01)*
***A61Q 3/02** (2006.01)*

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **17.12.2008 FR 0858696**
**17.12.2008 FR 0858695**
**23.12.2008 FR 0859040**
**18.02.2009 US 153322 P**
**18.02.2009 US 153328 P**
**21.01.2009 US 202024 P**

(71) Demandeur: **L'Oréal**
**75008 Paris (FR)**

(72) Inventeurs:
- **Kergosien, Guillaume**
**92370, Chaville (FR)**
- **Leuridan, Frédéric**
**77170, Brie Comte Robert (FR)**

(74) Mandataire: **Le Coupanec, Pascale A.M.P. et al**
**Nony & Associés**
**3, rue de Penthièvre**
**75008 Paris (FR)**

# (54) Vernis à ongles comprenant au moins un alkyléther ou un ester de polysaccharide et au moins une résine végétale

(57) La présente invention a pour objet un vernis à ongles comprenant un milieu solvant constitué d'un ou plusieurs solvants d'origine végétale, au moins un alkyléther ou un ester de polysaccharide et au moins une résine végétale.

Elle a en outre pour objet un procédé cosmétique de maquillage ou de soin non thérapeutique des ongles comprenant l'application sur les ongles d'au moins une couche d'un vernis à ongles conforme à l'invention.

Elle a enfin pour objet un article souple comprenant en particulier dans une couche adhésive, au moins une résine végétale, au moins un plastifiant et de préférence au moins un solvant d'origine végétale, **caractérisé en ce que** le plastifiant est choisi parmi les esters d'acide caprylique et/ou caprique ou ester d'alcool caprylique et/ou caprique et notamment les triglycérides d'acide caprylique et/ou caprique

EP 2 198 844 A2

## Description

La présente invention a pour objet une composition de vernis à ongles comprenant un milieu solvant constitué d'un ou plusieurs solvants d'origine végétale, au moins un alkyléther ou un ester de polysaccharide et au moins une résine végétale.

Cette composition peut être appliquée sur les ongles d'êtres humains ou bien encore sur des faux ongles.

La présente invention se rapporte en outre à un procédé de maquillage et/ou de soin non thérapeutique des ongles correspondant.

La composition de vernis à ongles, colorée ou transparente, peut être employée comme base pour vernis ou « base-coat » en terminologie anglo-saxonne, comme produit de maquillage des ongles, comme composition de finition, encore appelée « top-coat », à appliquer sur le produit de maquillage des ongles ou bien encore comme produit de soin cosmétique des ongles.

De nos jours, il est préférable, pour des raisons d'allergie aux vernis, d'éviter l'utilisation de certains composés et de les remplacer par d'autres moins nocifs, comme par exemple des composés d'origine végétale.

Ainsi, le document WO 2006/056558 décrit des compositions de vernis à ongles comprenant un solvant exclusivement d'origine végétale à base, notamment, de dérivés d'huile de fusel.

De même, le document EP 0 676 451 vise des dispersions aqueuses de résines végétales (résine de shellac, gomme de sandaraque, élémis, dammars et copal) afin de remplacer les pseudo-latex de résines synthétiques.

Par ailleurs, dans la demande WO 2007/080172, il a été proposé un plastifiant de type carbonate d'origine végétale dans une composition pour vernis à ongles.

De même, le document US 5,066,484 décrit des vernis à ongles comprenant un plastifiant de type triglycérides.

Il est également décrit dans US 5,145,670 des compositions de vernis à ongles comprenant des di- ou triesters comme agent plastifiant, et présentant des propriétés cosmétiques améliorées.

De même, le document JP 2005 263656 enseigne une composition de vernis dentaire comprenant une résine végétale (gomme benjoin).

Par ailleurs, le document JP 2004 315479 vise des compositions de vernis à ongles contenant un monoglycéride d'acide caprylique comme agent plastifiant.

Il est courant d'utiliser, dans les compositions de vernis à ongles, des agents filmogènes pour obtenir, après dépôt sur l'ongle, un film résistant présentant une bonne tenue. Actuellement, la nitrocellulose reste encore le filmogène le plus couramment utilisé dans les vernis à ongles présentant des propriétés de tenue optimisées.

Cependant, ces vernis à ongles comprenant de la nitrocellulose présentent l'inconvénient de ne pas être stables à la chaleur et au stockage.

Il en résulte notamment un jaunissement de ces compositions au cours du temps.

Par ailleurs, l'introduction de quantités importantes de nitrocellulose conditionne la composition de la phase solvant organique du vernis à ongle qui doit alors être compatible avec ce filmogène.

Il est par ailleurs utile de rappeler que la formulation des vernis à ongles est délicate car la composition est un ajustement judicieux entre les différents constituants. Le changement d'un de ces constituants peut modifier considérablement les propriétés cosmétiques du vernis et le rendre impropre à la consommation.

La présente invention a précisément pour but de remédier aux inconvénients ci-dessus et de fournir une composition de vernis à ongles comprenant une teneur réduite en nitrocellulose, voire exempte de nitrocellulose, présentant une bonne stabilité dans le temps, et permettant la formation d'un film homogène, brillant et/ou non cassant.

Les inventeurs ont ainsi découvert qu'il était possible d'obtenir une telle composition comprenant moins de 5 % en matière sèche de nitrocellulose par rapport au poids total de la composition, voire exempte de nitrocellulose, en utilisant un milieu solvant constitué d'un ou plusieurs solvants d'origine végétale, au moins un alkyléther ou un ester de polysaccharide, et au moins une résine végétale.

Ainsi, un premier aspect de l'invention concerne un vernis à ongles comprenant un milieu solvant constitué d'un ou plusieurs solvants d'origine végétale, au moins un alkyléther ou un ester de polysaccharide, et au moins une résine végétale, **caractérisé en ce que** le milieu solvant est choisi parmi un mélange d'éthanol et d'acétate d'éthyle.

Selon un autre aspect, l'invention concerne un vernis à ongles comprenant un milieu solvant constitué d'un ou plusieurs solvants d'origine végétale, au moins un alkyléther ou un ester de polysaccharide et au moins une résine végétale, **caractérisé en ce que** la résine végétale est choisie parmi la colophane et ses dérivés, les résines terpéniques, la gomme sandaraque, les dammars, l'élémi, les copals, le benjoin, la gomme mastic et leurs mélanges.

Un tel vernis à ongles selon ces aspects, permet notamment une bonne stabilité dans le temps et d'obtenir un film solide, voire dur, brillant, adhérent et/ou non cassant.

Selon encore un autre aspect, l'invention a pour objet un procédé cosmétique de maquillage ou de soin non thérapeutique des ongles comprenant l'application sur les ongles d'au moins une couche d'un vernis à ongles telle que défini précédemment.

Selon encore un autre de ses aspects, l'invention a pour objet l'utilisation d'un vernis à ongles tel que défini

précédemment pour obtenir, après dépôt sur l'ongle, un film homogène, brillant et non cassant.

Le vernis à ongles selon l'invention comprend un milieu cosmétiquement acceptable, c'est-à-dire un milieu non toxique et susceptible d'être appliqué sur les matières kératiniques d'êtres humains, en particulier les ongles.

La présente invention concerne encore un support synthétique maquillé comprenant un maquillage obtenu par le procédé de l'invention.

Selon encore un autre aspect, la présente invention concerne un vernis à ongles comprenant au moins un ester d'acide et/ou d'alcool caprylique/caprique, au moins un dérivé de polysaccharide n'ayant pas subi de réaction avec des oxydes d'alkylène, notamment un alkyléther ou un ester de polysaccharide et au moins une co-résine, notamment une résine végétale, l'ester d'acide et/ou d'alcool caprylique/caprique répondant à l'une des formules (I) à (X) suivantes :

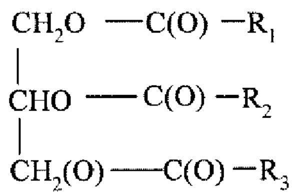
(I)

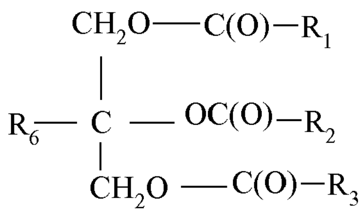
(II)

$F_1$-C(O)-O$CH_2$-C$(CH_3)_2$$CH_2$O-C(O)-$R_2$ (III)

$R_1$-C(O)-O$(Y)_d$-C(O)-$R_2$ (IV)

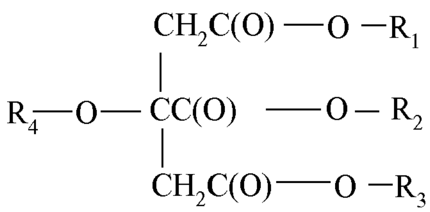
(V)

$R_7$-O-C(O)-$R_1$ (VI)

$CH_3$-$(CH_2)_5$-CH$(CH_3)$O-C(O)-$R_1$ (VII)

$R_6$-O-C(O)-$R_1$ (VIII)

$R_7$-O-C(O)-$R_6$ (IX)

$R_7$-O-C(O)-$(CH_2)_8$-C(O)-O-$R_8$ (X)

dans lesquelles :

$R_1$, $R_2$, $R_3$, identiques ou différents, représentent l'atome d'hydrogène ou une chaîne alkyle saturée, linéaire ou ramifiée, de préférence linéaire, à 7 ou 9 atomes de carbone,

$R_4$ représente l'atome d'hydrogène ou un groupement (Alk)-C(CO)-, dans lequel (Alk) représente une chaîne alkyle linéaire ou ramifiée comprenant entre 1 et 5 atomes de carbone,

$R_6$ représente une chaîne alkyle linéaire ou ramifiée, substituée ou non, comprenant entre 1 et 18 atomes de carbone, en particulier entre 1 et 10, ou une chaine alcényle linéaire ou ramifiée, substituée ou non, comprenant entre 2 et 18 atomes de carbone, en particulier entre 2 et 10,

Y est un groupe ethoxy, isopropoxy ou propoxy,

d est compris entre 1 et 1000, en particulier compris entre 1 et 15, en particulier égal à 1, et

$R_7$, $R_8$, identiques ou différents, représentent une chaîne alkyle saturée, linéaire

ou ramifiée, de préférence linéaire, à 8 ou 10 atomes de carbone.

Selon un mode de réalisation particulier, l'ester d'acide et/ou d'alcool caprylique/caprique est un triglycéride d'acide caprylique et/ou caprique.

Selon cet aspect, le vernis à ongles présente d'excellentes propriétés cosmétiques, parmi lesquelles on peut citer l'absence d'irritation des ongles, une bonne application, l'obtention d'un film homogène d'excellente brillance, un temps de séchage du film rapide, mais aussi une bonne adhérence sur la surface du support, une certaine flexibilité et une bonne résistance du film, par exemple en vue d'éviter les craquelures et son écaillement, et permet la réduction, voire l'élimination des plastifiants usuels.

Un vernis à ongles selon cet aspect permet notamment une bonne stabilité dans le temps et d'obtenir un film solide, brillant et/ou non cassant.

Selon un mode de réalisation particulier, la co-résine est une résine végétale telle que définie ci-après.

Selon encore un autre mode de réalisation particulier, le dérivé de polysaccharide n'ayant pas subi de réaction avec des oxydes d'alkylène est un alkyléther ou ester de polysaccharide tel que défini ci après.

Selon encore un autre de ses aspects, l'invention concerne un vernis à ongles comprenant (i) au moins un ester d'acide et/ou d'alcool caprylique/caprique, notamment répondant à l'une des formules (I) :

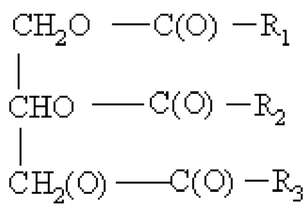

où $R_1$, $R_2$ et $R_3$ sont tels que définis ci-dessus,

ou (II) à (X) telles que définies ci-dessus, (ii) au moins un dérivé de polysaccharide n'ayant pas subi de réaction avec des oxydes d'alkylène, notamment un alkyléther ou ester de polysaccharide et (iii) au moins une co-résine, notamment une résine végétale, et comprenant moins de 5 % en matière sèche de nitrocellulose par rapport au poids total du vernis à ongles, voire exempt de nitrocellulose.

Selon un de ses aspects, le vernis à ongles présente une texture liquide ou gélifiée ; il diffère en particulier d'un patch ou d'un article souple comprenant au moins une couche de matériau polymérique et une couche d'adhésif, destiné à être collé et ajusté grâce à ses propriétés de déformabilité sur l'ongle.

Autrement dit, le vernis à ongles selon l'invention est typiquement appliqué sous forme de couches superposées à la surface des ongles ou des faux ongles à maquiller, par exemple à l'aide d'un pinceau.

Le vernis à ongles selon la présente invention peut prendre différentes formes; soit une forme classique de vernis à ongles, c'est-à-dire qu'il présente une texture liquide ou gélifiée comme exposé ci-dessus, soit une forme d'article s'apposant et s'ajustant sur les ongles, comme exposé ci-après.

La présente invention vise précisément à proposer un mode de maquillage et/ou de soin des ongles ou des faux ongles présentant un pouvoir collant, dont la composition permette une souplesse et une capacité d'élongation suffisantes au moment de l'application.

L'article souple selon l'invention comprend en particulier dans une couche adhésive au moins une résine végétale, au moins un plastifiant et de préférence au moins un solvant d'origine végétale, **caractérisé en ce que** le plastifiant est choisi parmi les esters d'acide caprylique et/ou caprique ou ester d'alcool caprylique et/ou caprique et

notamment les triglycérides d'acide caprylique et/ou caprique.

L'article souple peut notamment comprendre une couche polymérique et une couche adhésive.

Selon un mode de réalisation particulier, la résine végétale, le plastifiant et l'éventuel solvant d'origine végétale sont compris dans la couche adhésive.

Dans une variante, l'article peut comprendre une superposition de couches polymériques.

Dans une autre variante, l'article peut comprendre une ou plusieurs couches adhésives en sus de la ou des couches polymériques.

Selon un autre aspect, la présente invention concerne un produit de maquillage et/ou de soin des ongles et/ou faux ongles comprenant, dans un conditionnement sensiblement étanche à l'air, au moins un article conforme à l'invention, le conditionnement étant tel que l'article s'y trouve conservé sous une forme partiellement sèche.

Cet article souple est nommé pour des raisons de simplicité, dans la suite de la description, « article souple partiellement sec ».

Selon cet aspect de l'invention, l'article souple partiellement sec n'acquiert un aspect totalement sec, et donc sa forme définitive, qu'après application sur l'ongle et séchage, par simple exposition à l'air ambiant.

Selon un autre aspect, la présente invention concerne un procédé de maquillage et /ou de soin des ongles et/ou faux ongles comprenant au moins une étape consistant à appliquer sur un ongle naturel et/ou faux ongle, un article souple selon l'invention, qu'il soit considéré à l'état sec ou à l'état partiellement sec.

Selon un mode de réalisation, l'article souple conforme à la présente invention peut se présenter sous diverses formes telles qu'une étoile, un carré, un rond, etc.

Au sens de la présente invention, le terme « partiellement sec » entend qualifier le fait que l'article obtenu après formation du film n'est pas totalement exempt du solvant résiduel. En particulier, il possède une teneur en matière sèche supérieure à 80 %, plus particulièrement supérieure à 85 % et inférieure à 95 % en poids par rapport à son poids total.

Par opposition, l'article souple selon l'invention, objet du premier aspect de l'invention mentionné ci-dessus, est nommé « article souple à l'état sec ».

## 1- COMPOSITION DE VERNIS A ONGLES SOUS FORME LIQUIDE OU GELIFIEE

### Solvant d'origine végétale

Selon un mode de réalisation particulier, un vernis à ongles selon l'invention peut comprendre un milieu solvant constitué d'un ou plusieurs solvants d'origine végétale.

Autrement dit, selon ce mode de réalisation, la composition est alors complètement exempte de solvants d'origine non végétale.

Au sens de l'invention, on entend par « solvant d'origine végétale », un solvant que l'on obtient directement à partir de végétaux et n'ayant subi aucune modification chimique après extraction.

Les solvants d'origine végétale conformes à l'invention sont de préférence choisis parmi les alcools et/ou les acétates et/ou les lactates.

Selon un mode de réalisation préféré, le solvant d'origine végétale est notamment choisi parmi l'éthano et/ou l'acétate d'éthyle et/ou le lactate d'éthyle.

De préférence, il s'agira d'un mélange d'éthanol et d'acétate d'éthyle.

L'éthanol exclusivement d'origine végétale est largement disponible auprès de nombreux fournisseurs, notamment auprès de la société TEREOS. L'acétate d'éthyle d'origine végétale est disponible auprès de la société Rhodia. Le lactate d'éthyle est disponible auprès de la société REACT-NTI sous la référence ENVIROSOLVE SILVER.

Selon un mode particulier de réalisation, les compositions cosmétiques selon l'invention comprennent de 1 à 99 % de milieu solvant en poids, notamment de 40 à 90 % en poids, en particulier de 60 à 80 % en poids par rapport au poids total de la composition.

### Alkyléther ou ester de Polysaccharide

Selon un mode de réalisation, la composition de vernis à ongles selon l'invention comprend un alkyléther ou ester de polysaccharide.

Par « ester ou alkyléther de polysaccharide », on désigne au sens de l'invention un polysaccharide formé de motifs répétitifs comportant au moins deux cycles identiques ou différents et présentant un degré de substitution par unité de saccharide compris entre 1,9 et 3 de préférence compris entre 2,2 et 2,9, et plus particulièrement entre 2,4 et 2,8. On désigne par substitution la fonctionnalisation des groupements hydroxyles en fonctions esters et/ou alkyléthers, et/ou la fonctionnalisation des groupements carboxyliques en fonctions esters.

Autrement dit, l'invention désigne un polysaccharide, partiellement ou totalement substitué par des groupements esters et/ou alkyléthers.

Les groupements hydroxyles peuvent de préférence être substitués par des fonctions ester et/ou alkyléther en $C_2$-$C_4$.

Les unités de saccharides peuvent notamment être choisies parmi le mannose, le galactose, le glucose, le furanose, le rhamnose, l'arabinose, le xylose, le fucose, l'acide glucuronique, l'acide mannuronique, et l'acide galacturonique.

Parmi les composés appropriés, on peut citer les esters et/ou alkyléthers de cellulose dont le motif est constitué de glucose, les esters et/ou alkyléthers de guar ou de caroube dont les motifs sont constitués de galactose et de mannose, les esters et/ou alkyléthers de konjac dont le motif est constitué de glucose et de mannose, et les esters et/ou alkyléthers de tamarin dont le motif est constitué de galactose, de xylose, et de glucose.

On choisira notamment de préférence les acétobutyrates de cellulose, les acétopropionates de cellulose, les éthylcelluloses, et les éthylguars.

Comme ester ou alkyléther de polysaccharide, on peut notamment citer ceux vendus sous les dénominations commerciales AQUALON par la société HERCULES, ETHOCEL par la société DOW, et sous les dénominations CAP et CAB par la société EASTMAN.

Plus particulièrement, l'alkyléther ou l'ester de polysaccharide est choisi parmi les éthylguars, les éthylcelluloses, les acétopropionate, ou les acétobutyrates de cellulose.

La composition cosmétique selon la présente invention peut comprendre au moins une, et en particulier au moins deux alkyléthers et/ou esters de polysaccharide.

Selon un mode particulier de réalisation, le vernis à ongles selon l'invention comprend au moins 1 % en poids d'ester ou alkyléther de polysaccharide, de préférence au moins de 2 %, voire au moins 3 % en poids par rapport au poids total du vernis à ongles.

Selon un mode particulier de réalisation, le vernis à ongles selon l'invention comprend de 1 à 60 % en poids, notamment de 2 à 30 % en poids, en particulier de 4 à 10 % en poids d'ester ou alkyléther de polysaccharide par rapport au poids total du vernis à ongles.

**<u>Résine végétale</u>**

Selon un mode de réalisation, un vernis à ongles conforme à l'invention comprend au moins une résine végétale.

Les résines végétales conformes à l'invention sont de préférences choisies parmi :

- la colophane ou ses dérivés, les résines terpéniques, et
- la gomme sandaraque, les dammars, l'élémi, les copals, le benjoin, la gomme mastic.

La colophane est un mélange comprenant majoritairement des acides organiques appelés acides de colophane (principalement des acides de type abiétique et de type pimarique).

Il existe trois types de colophane : la colophane (« *gum rosin* ») obtenue par incision sur les arbres vivants, la colophane de bois (« *wood rosin* ») qui est extraite des souches ou du bois de pins, et l'huile de tall (« *tall oil rosin* ») qui est obtenue d'un sous-produit provenant de la production du papier.

Les dérivés de colophane peuvent être issus en particulier de la polymérisation, de l'hydrogénation et/ou de l'estérification (par exemple avec des alcools polyhydriques tels que l'éthylène glycol, le glycérol, le pentaérhytritol) des acides de colophanes. On peut citer par exemple les esters de colophanes commercialisés sous la référence FORAL 85, PENTALYN H et STAYBELITE ESTER 10 par la société HERCULES ; SYLVATAC 95 et ZONESTER 85 par la société ARIZONA CHEMICAL ou encore UNIREZ 3013 par la société UNION CAMP.

Les résines terpéniques sont typiquement issues de la polymérisation d'au moins un monomère choisi parmi $\alpha$-pinène, le $\beta$-pinène, le limonène, et leurs mélanges. Ces résines peuvent présenter un poids moléculaire allant de 300 à 2000 g/mol. De telles résines sont commercialisées par exemple sous la dénomination PICCOLYTE A115 et S 125 par la société Hercules, ZONAREZ 7100 ou ZONATAC 105 LITE par la société ARIZONA Chem.

La composition cosmétique selon la présente invention peut comprendre au moins une, et en particulier au moins deux résines végétales.

Selon un mode particulier de réalisation préféré, les résines végétales sont plus particulièrement de la gomme de sandaraque, le benjoin, ou l'élémi.

Le vernis à ongles selon l'invention comprend de 1 à 60 % en poids, notamment de 10 à 50 % en poids, en particulier de 15 à 35 % en poids de polymères filmogène par rapport au poids total de la composition.

De manière avantageuse, le vernis à ongles comprend moins de 5 % d'eau en poids, voire moins de 3 %, voire moins de 1 % par rapport au poids total du vernis à ongles, voire en est complètement dépourvu.

Selon un mode de réalisation de l'invention, une composition de vernis à ongles selon l'invention comprend moins de 5 % en poids de matière sèche de nitrocellulose par rapport au poids total de la composition, de préférence

moins de 3 % en poids, de préférence moins de 1,5 % en poids, de préférence moins de 1 % en poids.

Selon un autre mode de réalisation de l'invention, la composition de vernis à ongles selon l'invention est totalement dépourvue de nitrocellulose.

Selon un mode de réalisation de l'invention, un vernis à ongles comprend au moins un ester ou alkyléther de polysaccharide, notamment l'éthylguar ou l'acétobutyrate de cellulose, au moins un résine végétale, notamment la gomme de benjoin, la gomme élémi ou la résine de sandaraque et au moins un milieu solvant constitué d'un mélange d'éthanol et d'acétate d'éthyle.

**Dérivé de polysaccharide n'ayant pas subi de réaction avec des oxydes d'alkylène**

Selon un autre mode de réalisation de l'invention, la composition de vernis à ongles comprend un dérivé de polysaccharide n'ayant pas subi de réaction avec des oxydes d'alkylène.

Par « polysaccharide n'ayant pas subi de réaction avec des oxydes d'alkylène», on désigne au sens de l'invention un dérivé de polysaccharide formé de motifs répétitifs comportant au moins deux cycles identiques ou différents et n'ayant pas subi de réaction avec des oxydes d'alkylène tels que l'oxyde d'éthylène ou l'oxyde de propylène.

On cite à titre de dérivé de polysaccharide n'ayant pas subi de réaction avec des oxydes d'alkylène, outre les alkyléthers et esters de polysaccharide décrits précédemment, la nitrocellulose.

Comme dérivé de polysaccharide n'ayant pas subi de réaction avec des oxydes d'alkylène, on peut notamment citer celui vendu sous la dénomination commerciale NITROCELLULOSE par la société BERGERAC.

La composition cosmétique selon la présente invention peut comprendre au moins un, et en particulier au moins deux dérivés de polysaccharide n'ayant pas subi de réaction avec des oxydes d'alkylène conformes à l'invention.

Selon un mode particulier de réalisation, le vernis à ongles selon l'invention comprennent de 1 à 60 % en poids, notamment de 2 à 30 % en poids, en particulier de 4 à 10 % en poids de dérivé de polysaccharide n'ayant pas subi de réaction avec des oxydes d'alkylène par rapport au poids total du vernis à ongles.

**Co-résine**

Selon un mode de réalisation particulier, la composition de vernis à ongles selon l'invention comporte une co-résine.

Cette co-résine peut de préférence être, outre les résines végétales décrites précédemment, un ester d'acide gras tel que les résines alkydes décrites ci-après ou encore être une résine d'origine végétale choisie parmi:

- la gomme arabique, la gomme de guar, les dérivés du xanthane, la gomme de karaya ;
- les alginates, les carraghénanes, les ulvanes et autre colloïdes algaux ;
- les caprolactames, les pullulanes, les pectines, la mannane, les galactomannanes, les glucomannanes et leurs dérivés.

Les résines alkydes contribuent à la formation d'un film sur l'ongle et permettent d'améliorer la brillance ainsi que l'adhérence du film.

Les résines alkydes utilisées selon l'invention sont des polyesters comprenant des chaînes hydrocarbonées d'acides gras. De telles résines sont notamment décrites dans l'Encyclopedia of Chemical Technology - Kirk-Othmer, 4ème édition, volume 2, pages 53 à 63 dont le contenu est incorporé à titre de référence dans la présente demande. Ces résines sont obtenues par polymérisation de polyols et de polyacides ou de leur anhydride correspondant, en présence d'acides gras. Ces acides gras peuvent être employés tels quels ou bien sous forme de triglycérides d'acides gras ou bien encore présents dans les huiles lors de la synthèse de la résine alkyde.

Du fait de la présence de chaînes hydrocarbonées d'acides gras dans la résine alkyde, on définit couramment les résines alkydes par leur longueur en huile. Ainsi, on entend selon la présente demande par longueur en huile d'une résine alkyde, le pourcentage pondéral en chaînes hydrocarbonées d'acide gras présent dans la résine alkyde.

Comme polyols pouvant être employés pour la synthèse de résine alkyde, on peut citer le pentaérythritol, le triméthylol propane, le triméthylol éthane, le néopentyl glycol, le propylène glycol, l'éthylène glycol, l'hexane diol-1,6, le butanediol-1,4, le diéthylène glycol et en particulier le glycérol.

Comme polyacide ou anhydride, on peut notamment citer l'acide isophtalique, l'acide téréphtalique, l'anhydride trimellitique, l'anhydride maléique, l'acide adipique, l'acide fumarique, l'acide azélaïque, l'acide sébacique et en particulier l'anhydride phtalique.

Les acides gras utilisés pour l'obtention des résines alkydes présentes dans les compositions selon la présente invention répondent à la formule R-COOH, dans laquelle R désigne un radical hydrocarboné, saturé ou insaturé, ayant de 7 à 45 atomes de carbone environ, de préférence de 9 à 35 atomes de carbone, avantageusement de 15 à 35 atomes de carbone et mieux de 15 à 21 atomes de carbone.

Comme acide gras, on peut notamment citer l'acide palmitique, l'acide stéarique, l'acide oléique, l'acide ricinoléique, l'acide linoléique, l'acide linolénique et en particulier l'acide caprique.

Les acides gras sont présents dans la majorité des huiles d'origine naturelle, notamment sous la forme de triglycérides. Les triglycérides d'acides gras sont des esters résultant de la réaction des trois fonctions alcools de la glycérine sur des acides gras, ces acides gras pouvant être identiques ou différents. Les huiles d'origine naturelle peuvent donc être utilisées pendant la polymérisation. Elles peuvent être choisies parmi l'huile de lin, l'huile de bois de chine, l'huile de oititica, l'huile de soja, l'huile de tournesol, l'huile de carthame, l'huile de ricin, l'huile de coco (coprah), l'huile d'olive, l'huile de palme, l'huile de colza, l'huile d'arachide et le tallol (dite aussi « tall oil »).

Comme résines alkydes utilisables selon l'invention, on peut notamment citer celles vendues sous les dénominations « BECKOSOL ODE 230 70$^{E}$ » par la société Dainippon Ink & Chem (copolymère anhydride phtalique/glycérol/glycidyl decanoate dans l'acétate d'éthyle à 70 %), «NECOWEL 581® » (50 % en huile de soja), « NECOWEL 585® » (20 % en huile de tournesol), « NECOWEL 580® » (20 % en huile de tournesol), « NECOWEL 586 N® » (50 % en huile de soja), « NECOWEL EP 1161® » (50 % en huile de soja), « NECOWEL EP 1213® » (20 % en huile), « NECOWEL EP 2009® » (32 % en huile de tournesol), « NECOWEL EP 2019® » (20 % en huile), « NECOWEL EP 2275® » (35 % en huile), « NECOWEL EP 2329® » (34 % en huile), « NECOWEL EP 3016® » (30 % en huile) par la société ASHLAND, « URADIL XP 515 AZ® » (73 % en tallol), « URADIL XP 516 AZ® » (63 % en tallol) par la société DSM RESINS.

Selon un mode de réalisation préféré, la co-résine est du Beckosol ODE 230 70E (copolymère anhydride phtalique/glycérol/glycidyl decanoate dans l'acétate d'éthyle à 70 %).

La co-résine présente dans la composition selon l'invention peut prendre la forme d'un mélange de plusieurs résines.

Selon un mode particulier de réalisation, les vernis à ongles selon l'invention comprennent de 1 à 60 % en poids, notamment de 2 à 40 % en poids, en particulier de 4 à 30 % en poids de co-résine par rapport au poids total du vernis à ongles.

<u>**Ester d'acide et/ou d'alcool caprylique et/ou caprique**</u>

Selon un mode de réalisation, un vernis à ongles selon l'invention peut en outre comprendre un ester d'acide et/ou d'alcool caprylique et/ou caprique, ou autrement dit un ester d'acide caprylique et/ou caprique, un ester d'alcool caprylique et/ou caprique ou leur mélange, à titre de plastifiant, ledit ester étant d'origine végétale. On entend par acide ou alcool caprylique et/ou caprique des acides carboxyliques ou alcools linéaires ou ramifiés, de préférence linéaires, ayant 8 ou 10 carbones.

Au sens de la présente invention caprylique et/ou caprique est synonyme de caprylique/caprique.

Au sens de l'invention, on entend par « ester d'origine végétale », un ester que l'on obtient à partir de matières premières végétales ou d'origine végétale.

Cet ester peut répondre à l'une des formules (I) à (X) suivantes :

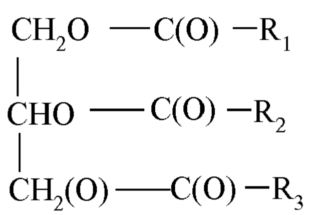

(I)

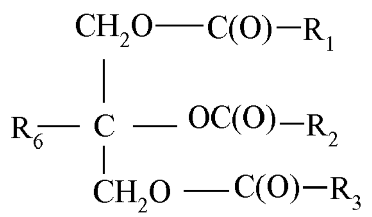

(II)

$R_1$-C(O)-OCH$_2$-C(CH$_3$)$_2$CH$_2$O-C(O)-$R_2$ (III)

$R_1$-C(O)-O(Y)$_d$-C(O)-$R_2$ (IV)

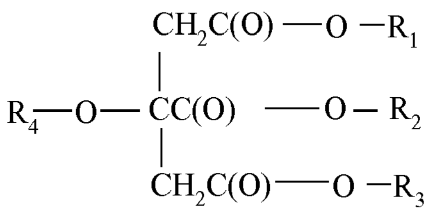

(V)

$R_7$-O-C(O)-$R_1$ (VI)

$CH_3$-$(CH_2)_5$-CH($CH_3$)O-C(O)-$R_1$ (VII)

$R_6$-O-C(O)-$R_1$ (VIII)

$R_7$-O-C(O)-$R_6$ (IX)

$R_7$-O-C(O)-$(CH_2)_8$-C(O)-O-$R_8$ (X)

dans lesquelles :

$R_1$, $R_2$, $R_3$, identiques ou différents, représentent l'atome d'hydrogène ou une chaîne alkyle saturée, linéaire ou ramifiée, de préférence linéaire, à 7 ou 9 atomes de carbone,
$R_4$ représente l'atome d'hydrogène ou un groupement (Alk)-C(CO)-, dans lequel (Alk) représente une chaîne alkyle linéaire ou ramifiée comprenant entre 1 et 5 atomes de carbone,
$R_6$ représente une chaîne alkyle linéaire ou ramifiée, substituée ou non, comprenant entre 1 et 18 atomes de carbone, en particulier entre 1 et 10, ou une chaine alcényle linéaire ou ramifiée, substituée ou non, comprenant entre 2 et 18 atomes de carbone, en particulier entre 2 et 10.
Y est un groupe ethoxy, isopropoxy ou propoxy, et
d est compris entre 1 et 1000, en particulier compris entre 1 et 15, en particulier égal à 1.
$R_7$, $R_8$, identiques ou différents, représentent une chaîne alkyle saturée, linéaire ou ramifiée, de préférence linéaire, à 8 ou 10 atomes de carbone.

L'ester conforme à l'invention peut également comprendre un mélange de différents esters définis ci-dessus.

De tels esters sont commercialement disponibles, par exemple auprès de la société Cognis ou RTD Hallstar.

De préférence, de tels esters sont choisis parmi les esters d'acide caprylique/caprique tels que mentionnés ci-dessus. De préférence, de tels esters sont choisis parmi les composés de formule (I), (VI), (VII) et (VIII). De préférence, de tels esters sont choisis parmi les esters d'acide caprylique/caprique linéaires.

Selon un mode de réalisation préféré de l'invention, ce plastifiant peut notamment être un composé de formule (I) tel que défini ci-dessus, ou autrement dit un triglycéride d'acide caprylique et/ou caprique.

Selon un autre mode de réalisation préféré de l'invention, ce plastifiant peut notamment être un composé de formule (VII) tel que défini ci-dessus, ou autrement dit un caprylate et/ou caprate d'isooctyle.

Selon un mode particulier de réalisation, les compositions cosmétiques selon l'invention comprennent de 0,5 à 15 % en poids, notamment de 1 à 10 % en poids, en particulier de 2 à 8 % en poids d'ester d'acide et/ou d'alcool caprylique et/ou caprique par rapport au poids total de la composition.

**Phase grasse liquide**

Les vernis à ongles conformes à la présente invention peuvent en outre comprendre une phase grasse liquide, de préférence d'origine naturelle. Conviennent ainsi à l'invention les huiles d'origine naturelle, qu'elles aient été ou non soumises à des opérations de transformation comme la transestérification ou l'hydrogénation ou un fractionnement.

On entend par huile, tout corps gras sous forme liquide à température ambiante (20-25 °C) et à pression atmosphérique. La phase grasse liquide peut, également, contenir outre les huiles, d'autres composés solubilisés dans les huiles tels que des agents gélifiants et/ou structurants.

A titre d'exemples d'huiles convenant à la mise en oeuvre de la présente invention, on peut mentionner les glycols d'origine naturelle tels que le glycérol ou le propylène glycol, les éthers de glycols d'origine naturelle, ainsi que les huiles d'origine naturelle choisies parmi des huiles comprenant au moins un acide gras choisi parmi l'acide caprylique,

l'acide caprique, l'acide laurique, l'acide myristique, l'acide palmitique, l'acide stéarique, l'acide oléique, l'acide ricinoléique, l'acide linoléique, l'acide linolénique, l'acide arachidique, l'acide gadoléique, l'acide béhénique, l'acide érucique, l'acide barssidique, l'acide cétoléique, l'acide lignocérique, l'acide nervonique, et un mélange de ceux-ci.

Le vernis à ongles selon la présente invention peut comprendre au moins une, et en particulier au moins deux huiles.

Les huiles peuvent être des huiles hydrocarbonées d'origine végétale.

Au sens de la présente invention, on entend par « huile hydrocarbonée », une huile contenant principalement des atomes d'hydrogène et de carbone et éventuellement des atomes d'oxygène, d'azote, de soufre et/ou de phosphore.

Selon un mode particulier de réalisation, les compositions cosmétiques conformes à l'invention peuvent comprendre au moins une huile choisie parmi les huiles hydrocarbonées végétales suivantes : le myristate d'isopropyle commercialisé par STEARINERIE DUBOIS, les triglycérides de l'acide caprylique/caprique commercialisés par STEPAN ; l'huile hybride de Colza, l'huile liquide de graines coton, l'huile de mangue désodorisée protégée raffinée, la fraction liquide de beurre de karité protégé et l'huile de graines de canola raffinée commercialisées par KARLSHAMNS ; le Lipex Sheasoft et l'huile de graines coton commercialisés par KARSLSHAMNS ; l'huile d'amandes d'abricot désodorisée commercialisée par NESTLE ; l'huile d'amande douce commercialisée par SOETENAEY ; l'huile d'amande de pêche commercialisée par AARHUS UNITED ; l'huile de colza, l'huile de germes de maïs, l'huile d'olive, l'huile de pépins de raisin, l'huile de soja et l'huile de tournesol commercialisées par HUILERIES DE LAPALISSE et l'huile de noix commercialisée par SOETENAEY.

Outre les huiles précitées, les vernis à ongles conformes à la présente invention peuvent bien entendu comprendre au moins une autre matière grasse liquide sous réserve que celle-ci soit présente dans des quantités conformes aux exigences selon l'invention.

Comme huile hydrocarbonée, on peut notamment citer :

- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné, et
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$.

Les esters peuvent être notamment choisis parmi les esters, notamment d'acide gras comme par exemple :

- l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en $C_{12}$ à $C_{15}$, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
- les esters de polyol et les esters du pentaérythritol, comme le tétrahydroxystéarate/tétraisostéarate de dipentaérythritol ;
- les esters de dimères diols et dimères diacides tels que les Lusplan DD-DAS® et Lusplan DD-DA7®, commercialisés par la société NIPPON FINE CHEMICAL et décrits dans la demande FR 03-02809 déposée le 6 mars 2003 ;
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol ; et
- les carbonates de di-alkyle, les 2 chaînes alkyles pouvant être identiques ou différentes, tel que le dicaprylyl carbonate commercialisé sous la dénomination CETIOL CC®, par COGNIS.

Selon un mode particulier de réalisation, le vernis à ongles selon l'invention comprend de 0,1 à 20 % en poids, notamment de 1 à 10 % en poids, en particulier de 1 à 5 % en poids de phase grasse liquide par rapport au poids total du vernis à ongles.

**Polymère filmogène additionnel**

Le vernis à ongles selon l'invention peut comprendre, outre l'alkyléther ou l'ester de polysaccharide, un polymère filmogène dit additionnel.

On entend par « polymère filmogène » au sens de la présente invention, un polymère apte à former à lui seul ou en présence d'un agent auxiliaire de filmification, un film isolable, notamment continu et adhérent, sur un support, notamment sur les ongles.

Dans le vernis à ongles, on peut utiliser un seul ou plusieurs polymères filmogènes additionnels.

Ce polymère filmogène additionnel peut être choisi dans le groupe constitué par les polymères synthétiques, de type radicalaire ou de type polycondensat, les polymères d'origine naturelles, et leur mélanges.

Le polymère filmogène additionnel peut être choisi en particulier parmi les polyuréthanes, les polymères acryliques, les polymères vinyliques, les polyvinylbutyrals, les résines alkydes, les résines cétone/aldéhyde, les résines issues des produits de condensation d'aldéhyde tels que les résines arylsulfonamide formaldéhyde comme la résine toluène sulfonamide formaldéhyde, les résines aryl-sulfonamide époxy, les résines éthyl tosylamide, ou encore les polymères filmogènes siliconés.

Comme polymère filmogène additionnel, on peut notamment utiliser les résines toluène sulfonamide formaldéhyde « Ketjentflex MS80 » de la société AKZO ou « Santolite MHP », « Santolite MS 80 » de la société FACONNIER ou « RESIMPOL 80 » de la société PAN AMERICANA, la résine alkyde « BECKOSOL ODE 230-70-E » de la société DAINIPPON, la résine acrylique « ACRYLOID B66 » de la société ROHM & HAAS, la résine polyuréthane « TRIXENE PR 4127 » de la société BAXENDEN, la résine acétophénone/formaldéhyde commercialisée sous la référence Synthetic Resin SK par Degussa.

Le polymère filmogène additionnel peut être présent dans la composition de vernis à ongles selon l'invention en une teneur allant de 0,1 % à 30 % en poids, par rapport au poids total de la composition, de préférence allant de 0,5 % à 20 % en poids, et mieux de 1 % à 10 % en poids.

**Agent gélifiant**

La composition de vernis à ongles selon l'invention peut comprendre en outre un agent gélifiant.

Cet agent gélifiant peut en particulier être choisi parmi : les silices hydrophobes, telles que celles décrites dans le document EP-A-0 898 960, et par exemple commercialisées sous les références « AEROSIL R812® » par la société Degussa, « CAB-O-SIL TS-530® », « CAB-O-SIL TS-610® », « CAB-O-SIL TS-720® » par la société Cabot, « AEROSIL R972® », « AEROSIL R974® » par la société Degussa ; les argiles telles que la montmorillonite, les argiles modifiées telles que les bentones par exemple commercialisées sous la référence BENTONE 27 V (Elementis), l'hectorite stéaralkonium, la bentonite stéaralkonium.

La proportion totale en agent(s) gélifiant(s) dans les compositions selon l'invention peut aller de à 0,01 à 15 % en poids, par rapport au poids total de la composition, de préférence de 0,5 à 15 % et mieux de 0,5 à 10 % en poids.

**Matière colorante**

La composition de vernis à ongles selon l'invention peut, en outre, comprendre une ou des matières colorantes choisies parmi les colorants solubles, et les matières colorantes pulvérulentes comme les pigments, les nacres, et les paillettes bien connues de l'homme du métier.

Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, polyester, polyuréthane, polyéthylène téréphtalate, les céramiques, les alumines et recouvert ou non de substances métalliques comme l'aluminium, l'or, le cuivre, le bronze, ou d'oxydes métalliques comme le dioxyde de titane, l'oxyde de fer, l'oxyde de chrome, de pigments minéraux ou

organiques et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

On peut aussi utiliser des pigments à propriétés goniochromatiques, notamment à cristaux liquides ou multicouche.

Les colorants sont, par exemple, le rouge Soudan, le DC Red 17, le DC Green 6, le β-carotène, l'huile de soja, le brun Soudan, le DC Yellow 11, le DC Violet 2, le DC Orange 5, le jaune quinoléine.

La matière colorante peut également être choisie parmi les azurants optiques.

La composition peut comprendre, en outre des fibres éventuellement enrobées d'azurant optiques.

**Charge**

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

**Additifs**

La composition peut comprendre, en outre, d'autres ingrédients utilisés couramment dans les compositions cosmétiques et connus de l'homme du métier comme étant susceptibles d'être incorporés dans une composition de vernis à ongles.

De tels ingrédients peuvent être choisis parmi les huiles, les cires, les agents anti-radicaux libres, les agents d'étalement, les agents mouillants, les agents dispersants, les anti-mousses, les conservateurs, les tensioactifs, les parfums, les neutralisants, les stabilisants, les antioxydants, les actifs pouvant être choisis parmi les huiles essentielles, les filtres UV/solaires, les agents hydratants, les vitamines, les protéines, les céramides, les extraits végétaux , etc. et leurs mélanges.

Les compositions selon l'invention peuvent comprendre en outre à titre d'actifs, des agents de soin des ongles tels que des agents durcissants pour matières kératiniques, des actifs agissant sur la croissance de l'ongle comme par exemple le méthyl sulfonyle méthane et/ou des actifs pour traiter des affections diverses localisées au niveau de l'ongle, comme par exemple l'onychomycose.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au

moins une touffe de poils. Alternativement, l'applicateur se présente sous forme autre qu'un pinceau, par exemple sous forme d'une spatule ou d'un embout en mousse.

## II - ARTICLE SOUPLE

D'une manière générale, l'article conforme à la présente invention se présente sous la forme d'un film ou laminé.
Au sens de la présente invention, le terme « souple » qualifie une flexibilité suffisante de ce film, c'est-à-dire propice à des déformations mécaniques de type étirement pour l'ajuster à la surface d'un ongle.
Par ailleurs, il faut également comprendre que par « souple », on entend apte à se déformer non élastiquement de manière à se conformer au profil plus ou moins bombé de l'ongle.
Cette déformabilité est notamment caractérisée par le paramètre de déformation à la rupture $\varepsilon_r$ discuté ci-après. L'article selon l'invention se différencie notamment à ce titre d'un article de type faux ongle, qui se caractérise par une rigidité incompatible avec une telle déformation mécanique.
Une autre différence entre l'article conforme à l'invention et un faux ongle réside dans la sensibilité de cet article vis-à-vis des solvants organiques polaires du type acétone, ester et/ou alcool court, tels que les acétates d'alkyle, notamment l'acétate d'éthyle.
En effet, l'article selon l'invention peut être aisément éliminé par démaquillage à l'aide d'un dissolvant classique ou solvant, par opposition à un faux ongle qui se retire.
L'invention concerne aussi un article susceptible d'être démaquillé à l'aide d'un solvant choisi parmi l'acétone, les acétates d'alkyles tel que l'acétate d'éthyle, et leurs mélanges.
A tous ces égards, l'article souple selon l'invention se distingue d'une part des compositions liquides classiques type vernis à ongle en raison de sa structure partiellement sèche, et d'autre part des produits solides types faux ongles, par le fait que ledit article avant application est déformable mécaniquement et peut se démaquiller, contrairement à un faux ongle qui est apposé directement sur l'ongle puis limé pour être ajusté et ensuite directement enlevé de l'ongle afin de retrouver un ongle à l'état normal.
L'article selon l'invention peut être utilisé à des fins de maquillage, auquel cas il comprend des matières colorantes, ou à des fins de protection vis-à-vis d'un film de vernis. Dans cette alternative, la couche polymérique est généralement transparente.
L'invention a aussi pour objet un article souple destiné à être appliqué sur les ongles et/ou faux ongles pour leur maquillage et/ou leur soin tel que défini ci-dessus, comprenant en outre au moins une couche adhésive.
Ladite couche adhésive peut également comprendre en outre un plastifiant.
En particulier, l'article souple peut comporter en outre une pellicule de protection au contact de la première face de la couche adhésive, à enlever préalablement à la mise en place de l'article sur l'ongle. De préférence la face de la pellicule de protection au contact de la première face de la feuille est recouverte d'un matériau antiadhésif, notamment siliconé.
Selon un mode de réalisation particulier, l'article conforme à l'invention est revêtu sur ses deux faces avec une pellicule amovible, identique ou différente.
Selon un mode de réalisation, l'article souple selon la présente invention présente une épaisseur allant de 6 $\mu$m à 1 mm, en particulier de 10 $\mu$m à 500 $\mu$m, et encore plus particulièrement de 50 $\mu$m à 200 $\mu$m.
L'épaisseur visée s'entend comme étant l'épaisseur avant application sur l'ongle de l'intégralité de la structure indissociable à une ou plusieurs couches comprenant notamment la couche adhésive.
En particulier, l'épaisseur s'entend de l'épaisseur de l'ensemble des couches polymériques et adhésives.
En revanche, toute structure fixée de manière amovible à l'article souple, en particulier une pellicule de protection sur l'une ou l'autre des faces de l'article, en particulier, une pellicule siliconée sur la face adhésive de l'article, n'entre pas en ligne de compte dans la mesure de l'épaisseur.
L'article souple, et en particulier l'excès, peut être prédécoupé ou découpé, avant ou après son application, selon la taille et la forme désirée avec de petits ciseaux, un coupe ongles ou en grattant le film.
L'article souple ou article souple à l'état sec selon l'invention se présente notamment sous la forme d'un film non liquide qui peut se caractériser par un extrait sec élevé. En effet, la quantité de matière sèche dans cet article à l'état sec, à savoir une fois appliqué sur l'ongle ou le faux ongles, est supérieure ou égale à 95 % en poids par rapport au poids total de l'article. Autrement dit, la quantité de solvant volatil est inférieure ou égale à 5 % en poids par rapport au poids total de l'article.
Le produit de maquillage, selon le deuxième aspect évoqué précédemment comporte un article sous forme partiellement sèche. Dans ce cas particulier, le produit de maquillage est conditionné dans un réservoir comme par exemple une poche, souple ou non, suffisamment étanche pour lui préserver cet aspect partiellement sec. Ce n'est qu'au moment de son utilisation et par conséquent lors de sa mise en contact avec l'air, que le produit sèche totalement pour acquérir la teneur en matière sèche décrite précédemment, conforme à l'article conforme à l'invention.
Comme mentionné précédemment, dans un produit selon l'invention, l'article partiellement sec possède avantageusement une teneur en matière sèche supérieure à 80 %, notamment supérieure à 85 % et inférieure à 95 % en poids par rapport à son poids total. Un tel article, lorsqu'il est extrait du conditionnement d'un produit conforme à l'invention et exposé à l'air ambiant, acquiert un état sec tel que défini ci-dessus au terme de 24 heures.

De préférence, la quantité de matière sèche, communément appelée extrait sec des articles selon l'invention, est mesurée par échauffement de l'échantillon par des rayons infrarouges de 2 $\mu$m à 3,5 $\mu$m de longueur d'onde. Les substances contenues dans lesdits films qui possèdent une pression de vapeur élevée, s'évaporent sous l'effet de ce rayonnement. La mesure de la perte de poids de l'échantillon permet de déterminer l'extrait sec de l'article. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant :

> On dépose environ 10 g d'échantillon d'un article sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

$$\text{Extrait Sec} = 100 \text{ x (masse sèche/ masse humide).}$$

Reprise à l'eau

L'article selon l'invention peut être avantageusement caractérisé à l'état sec par une reprise à l'eau portée à 25 °C inférieure ou égale à 20 %, notamment inférieure ou égale à 16 %, et en particulier inférieure à 10 %.

Selon la présente demande, on entend par « reprise à l'eau », le pourcentage d'eau absorbé par l'article après 60 minutes d'immersion dans l'eau, à 25 °C (température ambiante). La reprise à l'eau est mesurée pour des morceaux d'environ 1 cm découpés dans l'article sec. Ils sont pesés (mesure de la masse Ml) puis immergés dans l'eau pendant 60 minutes ; immersion, le morceau de film est essuyé pour éliminer l'excédent d'eau en surface puis pesé (mesure de la masse M2). La différence M2 - M1 correspond à la quantité d'eau absorbée par le film.

La reprise à l'eau est égale à [(M2 - Ml) / Ml] x 100 et est exprimée en pourcentage de poids par rapport au poids du film.

Module de conservation E'

Par ailleurs, l'article selon l'invention est avantageusement un film ayant un module de conservation E' supérieur ou égal à 1 MPa, notamment allant de 1 MPa à 5000 MPa, en particulier supérieur ou égal à 5 MPa, notamment allant de 5 à 1000 MPa, et plus particulièrement supérieur ou égal à 10 MPa par exemple allant de 10 à 500 MPa à une température de 30 °C et une fréquence de 0,1 Hz.

La mesure du module de conservation est effectuée par DMTA (Dynamical and Mechanical Température Analysis ou Analyse dynamique et mécanique en température).

On effectue des essais de viscoélasticimétrie avec un appareil DMTA de Polymer TA Instruments (modèle DMA2980), sur un échantillon d'article. On découpe (par exemple à l'emporte-pièce) les éprouvettes. Celles-ci ont typiquement une épaisseur d'environ 150$\mu$m, une largeur de 5 à 10 mm et une longueur utile d'environ 10 à 15 mm. Les mesures sont effectuées à une température constante de 30 °C.

L'échantillon est sollicité en traction et en petites déformations (on lui impose par exemple un déplacement sinusoïdal de $\pm$ 8 $\mu$m) lors d'un balayage en fréquence, la fréquence allant de 0,1 à 20 Hz. On travaille ainsi dans le domaine linéaire, sous de faibles niveaux de déformation.

Ces mesures permettent de déterminer le module complexe E* = E' + iE" du film de composition testé, E' étant le module de conservation et E" le module dit de perte.

Déformation et/ou Energie à la rupture

Avantageusement, les articles selon l'invention, possèdent une déformation à la rupture $\varepsilon_r$ supérieure ou égale à 5 %, notamment allant de 5 à 500 %, de préférence supérieure ou égale à 15 %, notamment allant de 15 à 400 % et/ou une énergie à rupture par unité de volume Wr supérieure ou égale à 0,2 J/cm$^3$, notamment allant de 0,2 à 100

J/cm$^3$, de préférence supérieure à 1 J/cm$^3$, notamment allant de 1 à 50 J/cm$^3$.

La déformation à la rupture et l'énergie à rupture par unité de volume sont déterminées par des essais de traction effectués sur un film réticulé d'environ 200 μm d'épaisseur.

Pour effectuer ces essais, l'article est découpé en éprouvettes haltères de longueur utile 33 ±1 mm et de largeur utile 6 mm.

La section (S) de l'éprouvette est alors définie comme :

S = largeur x épaisseur (cm$^2$) ; cette section sera utilisée pour le calcul de la contrainte.

Les essais sont réalisés, par exemple, sur un appareil de traction commercialisé sous l'appellation Lloyd® LR5K. Les mesures sont réalisées à température ambiante (20 °C).

Les éprouvettes sont étirées à une vitesse de déplacement de 33 mm/min, correspondant à une vitesse de 100 % d'allongement par minute.

On impose donc une vitesse de déplacement et on mesure simultanément l'allongement ΔL de l'éprouvette et la force F nécessaire pour imposer cet allongement.

C'est à partir de ces données ΔL et F que l'on détermine les paramètres contraintes σ et déformation ε. Il est ainsi obtenu une courbe contrainte σ = (F/S) en fonction de la déformation ε = (ΔL/Lo) x 100, l'essai étant conduit jusqu'à rupture de l'éprouvette, Lo étant la longueur initiale de l'éprouvette. La déformation à la rupture $\varepsilon_r$ est la déformation maximale de l'échantillon avant le point de rupture (en %). L'énergie à rupture par unité de volume Wr en J/cm$^3$ est définie comme la surface sous cette courbe contrainte/déformation telle que :

$$Wr = \int_0^{\varepsilon_r} \sigma . \varepsilon . d\varepsilon$$

De préférence, l'article souple conforme à l'invention comprend plus de 80 %, de préférence plus de 90 %, de préférence plus de 95 % en poids de composés d'origine naturelle par rapport au poids total de l'article souple, de préférence l'article souple comprend exclusivement des composés d'origine naturelle.

## COUCHE ADHESIVE

L'article selon l'invention peut posséder une face externe adhésive si la couche polymérique n'est pas auto-adhésive. Une telle face adhésive est obtenue de façon générale grâce à la présence d'au moins une couche adhésive, **caractérisée en ce que** cette couche adhésive comprend au moins un matériau adhésif.

Par « matériau », on entend au sens de la présente invention un polymère ou un système polymérique pouvant comprendre un ou plusieurs polymères de natures différentes. Ce matériau adhésif peut en outre contenir un plastifiant.

Ce dernier doit présenter un certain pouvoir collant défini par ses propriétés viscoélastiques.

Les propriétés viscoélastiques d'un matériau sont classiquement définies par deux valeurs caractéristiques qui sont les suivantes :

- le module élastique qui représente le comportement élastique du matériau pour une fréquence donnée et qui est classiquement noté G',
- le module visqueux qui représente le comportement visqueux du matériau pour une fréquence donnée et qui est classiquement noté G".

Ces grandeurs sont notamment définies dans le « Handbook of Pressure Sensitive Adhesive Technology », 3d edition, D. Satas, chap. 9, p. 155 à 157.

De préférence, les matériaux adhésifs utilisables selon la présente invention présentent des propriétés viscoélastiques qui sont mesurées à une température de référence de 35 °C et dans un certain intervalle de fréquences.

Dans le cas de matériaux adhésifs sous forme de solution ou de dispersion de polymère dans un solvant volatil (tel que l'eau, un ester court, un alcool court, de l'acétone, etc), on mesure les propriétés viscoélastiques de ce matériau dans des conditions

dans lesquelles il présente une teneur en solvant volatil inférieure à 30 %, et, en particulier, une teneur en solvant volatil inférieure à 20 %.

On mesure en particulier le module élastique du matériau à trois fréquences différentes :

- à faible fréquence, soit à $2.10^{-2}$ Hz ;
- à une fréquence intermédiaire, soit à 0,2 Hz ;
- à haute fréquence, soit à 2 Hz, et
- le module visqueux à la fréquence de 0,2 Hz.

Ces mesures permettent d'évaluer l'évolution du pouvoir collant du matériau adhésif au cours du temps.
Ces propriétés viscoélastiques sont mesurées lors d'essais dynamiques sous sollicitations sinusoïdales de faible amplitude (petites déformations) réalisés à 35 °C sur une plage de fréquence allant de $2.10^{-2}$ à 20 Hz sur un rhéomètre de type « Haake RS50® »sous une sollicitation de torsion/cisaillement, par exemple en géométrie cône-plan (par exemple avec un angle du cône de 1°).
Avantageusement, ledit matériau adhésif répond aux conditions suivantes :

- G'(2 Hz, 35 °C) ≥ $10^3$ Pa, et
- G'(35 °C) ≤ $10^8$ Pa, en particulier, G'(35 °C) ≤ $10^7$ Pa,
- G' ($2.10^{-2}$ Hz, 35 °C) ≤ $3.10^5$ Pa,

dans lesquelles :

- G'(2 Hz, 35 °C) est le module élastique de cisaillement dudit matériau adhésif, mesuré à la fréquence de 2 Hz et à la température de 35 °C,
- G'(35 °C) est le module élastique de cisaillement dudit matériau adhésif, mesuré à la température de 35 °C, pour toute fréquence comprise entre 2. $10^{-2}$ et 2 Hz,
- G'($2.10^{-2}$ Hz, 35 °C) est le module élastique de cisaillement dudit matériau adhésif, mesuré à la fréquence de $2.10^{-2}$ Hz et à la température de 35 °C.

Dans une forme particulière de l'invention, le matériau adhésif répond également à la condition suivante :

- G"/G' (0,2 Hz, 35 °C) ≥ 0,35.

dans laquelle :

- G"(0,2 Hz, 35 °C) est le module visqueux de cisaillement dudit matériau adhésif, mesuré à la fréquence de 0,2 Hz et à la température de 35 °C,
- G'(0,2 Hz, 35 °C) est le module élastique de cisaillement dudit matériau adhésif, mesuré à la fréquence de 0,2 Hz et à la température de 35 °C.

Dans une forme particulière de l'invention, on a :

- G'(2 Hz, 35 °C) ≥ $5.10^3$ Pa, et en particulier, G'(2 Hz, 35 °C) ≥ $10^4$ Pa.

Dans une autre forme particulière de l'invention, on a :

- G'($2.10^{-2}$ Hz, 35 °C) ≤ $5.10^4$ Pa.

En particulier, les matériaux adhésifs selon l'invention répondent aux quatre conditions suivantes :

- G'(2 Hz, 35 °C) ≥ $10^4$ Pa, et
- G'(35 °C) ≤ $10^8$ Pa, en particulier G'(35 °C) ≤ $10^7$ Pa,
- G'($2.10^{-2}$ Hz, 35 °C) ≤ $5.10^4$ Pa, et
- G"/G'(0,2 Hz, 35 °C) ≥ 0,35.

Dans le cadre de la présente invention, le matériau adhésif comprend un ester d'acide et/ou d'alcool caprylique et/ou caprique en tant que plastifiant et une résine végétale telle que décrite dans le paragraphe I - composition de vernis à ongles sous forme liquide ou gélifiée. Il peut comprendre en outre un matériau adhésif additionnel.
Les matériaux adhésifs additionnels peuvent être choisis parmi les adhésifs de type « *Pressure Sensitive Adhesives* » (adhésifs sensibles à la pression) par exemple, comme ceux cités dans le « Handbook of Pressure Sensitive Adhesive Technology », 3d edition, D. Satas.
Les matériaux adhésifs additionnels peuvent en particulier être des polymères choisis parmi :

- les polyuréthannes,
- les polymères acryliques,
- les gommes butyliques, notamment parmi les polyisobutylènes,
- les polymères éthylène-acétate de vinyle,
- les polyamides éventuellement modifiés par des chaînes grasses,
- les adhésifs siliconés, notamment les copolymères de résine de silicone et de silicone fluide, tels que ceux commercialisés par Dow Corning sous la référence Bio-PSA,
- et leurs mélanges.

Il peut en particulier s'agir de copolymères adhésifs dérivant de la copolymérisation de monomères vinyliques avec des entités polymériques, comme par exemple, ceux décrits dans le brevet US 6,136,296. Sont également susceptibles de convenir à l'invention, les copolymères adhésifs décrits dans le brevet US 5,929,173 possédant un squelette polymérique, de Tg variant de 0 °C à 45 °C, greffés par des chaînes dérivant de monomères acryliques et/ou méthacryliques et possédant en revanche une Tg variant de 50 °C à 200 °C.

Ces matériaux adhésifs additionnels peuvent également être des polymères choisis parmi les copolymères blocs ou statistiques comprenant au moins un monomère ou une association de monomères dont le polymère résultant à une température de transition vitreuse inférieure à la température ambiante (25 °C), ces monomères ou associations de monomères pouvant être choisis parmi le butadiène, l'éthylène, le propylène, l'isoprène, l'isobutylène et leurs mélanges. Des exemples de tels matériaux sont les polymères blocs de type styrène-butadiène-styrène, styrène-(éthylène-butylène)-styrène, styrène-isoprène-styrène comme ceux vendus sous les dénominations commerciales « Kratori » de Shell Chemical Co. ou de « Vector® » de la société Dexco Polymers.

Les matériaux adhésifs additionnels sont par exemple choisis parmi les polyisobutylènes présentant une masse molaire relative Mv supérieure ou égale à 10 000 et inférieure ou égale à 150 000. En particulier, cette masse molaire relative est supérieure ou égale à 18 000 et inférieure ou égale à 150 000.

Comme produits commerciaux convenant particulièrement bien à la présente invention, on peut citer les polyisobutylènes de masses molaires relatives Mv respectives 40 000, 55 000 et 85 000 vendus sous les dénominations commerciales respectives « Oppanol B 10® », « Oppanol B 12® » et « Oppanol B 15® » par la société BASF, et leurs mélanges.

Selon un mode de réalisation particulier, le matériau adhésif additionnel comprend au moins un caoutchouc naturel et au moins une résine tackifiante non végétale.

Au sens de l'invention, on entend par caoutchouc naturel, un caoutchouc naturel que l'on obtient directement à partir de végétaux, et qui peut optionnellement avoir été modifié notamment par réaction chimique. De préférence, le caoutchouc naturel ne subit aucune modification chimique après extraction.

Selon un mode privilégié de l'invention, le caoutchouc naturel est obtenu après élimination de la phase aqueuse d'un latex naturel de polyisoprène, choisi de préférence parmi les latex d'hévéa, de Parthernium Argentatum (guayule), de solidago virgaurea minuta, de Taraxacum koksaghyz, de Gutta percha, de Gutta balata, de sapotillier (Manilkara zapota) et leurs mélanges.

De préférence, les latex naturels sont déprotéinisés, en particulier pour réduire les risques d'allergies.

A titre de résines tackifiantes *non végétales,* on peut citer les résines hydrocarbonées aliphatiques ou aromatiques, les résines phénoliques, les résines styréniques et coumarone-indène.

La couche adhésive comprend en outre un ester d'acide caprylique et/ou caprique ou un ester d'alcool caprylique et/ou caprique à titre de plastifiant tel que décrits ci-dessus. La couche adhésive peut en outre comporter des plastifiants additionnels, de préférence naturels ou d'origine naturelle.

## Plastifiants additionnels

Les plastifiants additionnels conformes à l'invention sont de préférences choisis parmi les composés éventuellement modifiés d'origine naturelle tels que les huiles comprenant au moins un acide gras choisi parmi l'acide caprique, l'acide laurique, l'acide myristique, l'acide palmitique, l'acide stéarique, l'acide oléique, l'acide rinoléique, l'acide linoléique, l'acide linolénique, l'acide arachidique, l'acide gadoléique, l'acide béhénique, l'acide érucique, l'acide brassidique, l'acide cétoléique, l'acide lignocérique, l'acide nervonique et un mélange de ceux-ci.

En particulier, les huiles convenant à la mise en oeuvre de l'invention sont choisies parmi les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées. Ces huiles sont notamment des triglycérides héptanoïques ou octanoïques, les huiles d'arachide, de babassu, de noix de coco, de pépins de raisin, de coton, de maïs, de germes de maïs, de graines de moutarde, de palme, de colza, de sésame, de soja, de tournesol, de germes de blé, de canola, d'abricot, de mangue, de ricin, de karité, d'avocat, d'olive, d'amande douce, d'amande de pêche, de noix, de noisette, de macadamia, de jojoba, de luzerne, de pavot, de potimarron, de courge, de cassis,

d'onagre, de millet, d'orge, de quinoa, de seigle, de carthame, de bancoulier, de passiflore, de rosier muscat, de beurre de karité, et leurs mélanges.

Selon un mode particulier de réalisation, les couches adhésives selon l'invention comprennent de 0,1 à 70 % en poids, notamment de 5 à 60 % en poids, et plus particulièrement de 10 à 50 % en poids de plastifiant par rapport au poids total de l'extrait sec de la couche adhésive, étant entendu que le plastifiant est constitué de l'ester d'acide caprylique et/ou caprique ou de l'ester d'alcool caprylique et/ou caprique et de tout autre éventuel plastifiant additionnel.

Selon un mode particulier de l'invention, la couche adhésive est composée d'au moins un caoutchouc naturel, éventuellement déprotéinisé, tel qu'un latex naturel de polyisoprène dépourvu de sa phase aqueuse, d'au moins une résine végétale parmi celles mentionnées ci-dessus, et d'au moins un plastifiant tel que défini ci-dessus.

Polysaccharides et dérivés de polysaccharides

Les polysaccharides ou dérivés de polysaccharide sont tels que décrits ci-dessus dans le paragraphe I - composition de vernis à ongles sous forme liquide ou gélifiée.

Selon un mode particulier de réalisation, la couche adhésive selon l'invention comprend de 0 à 60 % en poids, notamment de 5 à 40 % en poids, et plus particulièrement de 10 à 30 % en poids de polysaccharides ou dérivés de polysaccharides par rapport au poids total de l'extrait sec de la couche adhésive.

Le dérivé de polysaccharide préféré est l'éthylguar ou l'éthyl cellulose.

De manière avantageuse, l'article comprend de l'éthylguar dans une teneur allant de 10 à 20 % en poids par rapport au poids total de l'extrait sec de la couche adhésive.

De préférence, la couche adhésive conforme à l'invention comprend plus de 80 % en poids, de préférence plus de 90 % en poids, de préférence plus de 95 % en poids de composés d'origine naturelle par rapport au poids total de l'extrait sec de la couche adhésive.

En particulier, la couche adhésive peut être constituée exclusivement de composés d'origine naturelle.

La couche adhésive dans l'article conforme à l'invention est généralement sous la forme d'une couche ayant une épaisseur de 5 à 100 $\mu$m, en particulier de 10 à 80 $\mu$m, de préférence de 15 à 50 $\mu$m, de préférence de 20 à 30 $\mu$m.

**COUCHE POLYMERIQUE**

L'article selon la présente invention peut comporter au moins une couche polymérique qui peut comprendre au moins un latex naturel, éventuellement déprotéinisé, de préférence un latex naturel de polyisoprène.

En effet, il a été mis en évidence que l'utilisation de latex naturel et notamment de latex naturel de polyisoprène pour former la couche polymérique d'un film adhésif permettait de former des films à la fois souples et résistants, pouvant être étirés facilement lors de l'application sur les ongles.

Par « latex naturel », on entend dans le cadre de la présente invention un latex d'origine naturelle, à savoir que l'on obtient directement à partir de végétaux, et qui peut optionnellement avoir été modifié notamment par réaction chimique. De préférence, le « latex naturel » n'a subi aucune modification chimique.

Selon un mode de réalisation particulier, le latex naturel est distinct d'un caoutchouc naturel.

Selon un mode privilégié de l'invention, le latex naturel de polyisoprène est choisi parmi les latex d'hévéa, de Parthernium Argentatum (guayule), de solidago virgaurea minuta, de Taraxacum koksaghyz, de Gutta percha, de Gutta balata, de sapotillier (Manilkara zapota) et leurs mélanges.

De préférence, les latex sont déprotéinisés, en particulier pour réduire les risques d'allergies.

De préférence, la couche polymérique comprend un latex d'Hévéa, voire en est constituée.

Des latex d'hévéa conformes à l'invention sont disponibles auprès de la société SRI Hybrid sous la gamme Selatex, auprès de la société Getahindus sous la gamme Loprotex, auprès de la société Vystar Corporation sous la gamme Vytex. Des latex de guayule conformes à l'invention sont disponibles auprès de la société Yulex. Des latex de Gutta percha sont disponibles auprès de la société Rimpex Rubber.

De manière avantageuse, l'article comprend du latex naturel dans une teneur allant de 30 % à 100 %, de préférence 50 % à 100 %, et plus particulièrement de 70 % à 100 % en poids de latex naturel par rapport au poids total de l'extrait sec de la couche polymérique. (La couche polymérique peut en outre contenir des matières colorantes, des filtres solaires, d'autres filmogènes, des charges...).

De préférence, la couche polymérique conforme à l'invention comprend plus de 80 %, de préférence plus de 90 %, de préférence plus de 95 % en poids de composés d'origine naturelle par rapport au poids total de la couche polymérique, de préférence la couche polymérique comprend exclusivement des composés d'origine naturelle.

Selon un mode particulier de réalisation, l'article souple conforme à l'invention comprend une couche polymérique, laquelle comprend du latex d'hévéa, par exemple déprotéinisé, et une couche adhésive, laquelle comprend de la gomme sandaraque, du dammar, de l'élémi, des copals, du benjoin, de la gomme mastic, ou leurs mélanges, et au moins un plastifiant choisi parmi les esters ou des triglycérides d'acides caprylique/caprique, et éventuellement de

l'éthylguar et un latex naturel.

**Solvant d'origine végétale**

L'article selon l'invention peut comprendre un milieu solvant constitué d'un ou plusieurs solvants d'origine végétale.

Selon un mode particulier de réalisation, l'article souple ou article souple à l'état sec selon l'invention comprend de 1 à 20 % de milieu solvant en poids, notamment de 1 à 10 % en poids, en particulier de 1 à 5 % en poids par rapport au poids total de l'article souple.

**Polymère filmogène additionnel**

L'article selon l'invention peut comprendre un polymère filmogène additionnel tel que décrit dans le paragraphe ci-dessus, en particulier dans les mêmes teneurs.

**L'agent auxiliaire de filmification**

La couche polymérique peut comprendre en outre un agent auxiliaire de filmification qui peut être choisi parmi tous les composés connus de l'homme du métier comme étant susceptibles de remplir la fonction recherchée, et être notamment choisi parmi les agents plastifiants et les agents de coalescence du polymère filmogène.

En particulier, on peut citer les huiles hydrocarbonées telles que le parleam et les polyisobutènes.

On peut citer aussi à titre d'agent auxiliaire de filmification les plastifiants tel que décrits ci-dessus, y compris les esters d'acide caprylique et/ou caprique ou les esters d'alcool caprylique et/ou caprique également décrits ci-dessus.

On peut citer aussi les plastifiants ou agents de coalescence usuels, tels que: les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther, les esters de glycol, les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther, les esters d'acides notamment carboxyliques tels que les citrates, notamment le citrate de triéthyle, le citrate de tributyle, l'acétylcitrate de triéthyle, l'acétylcitrate de tributyle, l'acétylcitrate de triéthyl-2 hexyle ; les phtalates, notamment le phtalate de diéthyle, le phtalate de dibutyle, le phtalate de dioctyle, le phtalate de dipentyle, le phtalate de diméthoxyéthyle ; les phosphates, notamment le phosphate de tricrésyle, le phosphate de tributyle, le phosphate de triphényle, le phosphate de tributoxyéthyle ; les tartrates, notamment le tartrate de dibutyle ; les adipates ; les carbonates ; les sébaçates ; le benzoate de benzyle, l'acétylricinoléate de butyle, l'acétylricinoléate de glycéryle, le glycolate de butyle, le camphre, le triacétate de glycérol, le N-éthyl-o,p-toluènesulfonamide, les dérivés oxyéthylénés tels que les huiles oxyéthylénées, les huiles de silicone, et leurs mélanges.

Le type et la quantité d'agent plastifiant et/ou de coalescence peuvent être choisis par l'homme du métier sur la base de ses connaissances générales. Par exemple, la teneur en agent plastifiant et/ou de coalescence peut aller de 0,01 % à 20% et en particulier de 0,5 % à 10 % en poids par rapport au poids total de la couche polymérique.

Selon un mode de réalisation particulier, l'invention vise un article souple comprenant une couche polymérique comprenant du latex naturel distinct d'un caoutchouc naturel et une couche adhésive comprenant un polysaccharide ou dérivé de polysaccharide.

**Autres additifs**

**i) Pigments et colorants**

La couche adhésive et la couche polymérique de l'article selon l'invention peuvent comprendre notamment au moins une matière colorante, organique ou inorganique, notamment de type pigments ou nacres classiquement utilisée dans les compositions cosmétiques.

Par pigments, il faut comprendre des particules blanches ou colorées, minérales ou organiques, insolubles dans le milieu ou aqueux, destinées à colorer et/ou opacifier le film résultant.

Les pigments peuvent être présents à raison de 0,01 à 20 % en poids, notamment de 0,01 à 15 % en poids, et en particulier de 0,02 à 10 % en poids, par rapport au poids total de la première composition et/ou du film organique et/ou minéral.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et

l'hydrate de chrome.
Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-0 542 669, EP-A-0 787 730, EP-A-0 787 731 et WO-A-96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.
On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.
Les nacres peuvent plus particulièrement posséder une couleur ou un reflet jaune, rose, rouge, bronze, orangé, brun, or et/ou cuivré.

A titre illustratif, des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La première composition et/ou le film organique et/ou minéral selon l'invention peuvent comprendre également des colorants hydrosolubles ou liposolubles en une teneur allant de 0,01 à 10 % en poids, notamment allant de 0,01 à 5 % en poids par rapport au poids total de la première composition ou du film organique et/ou minéral. Les colorants liposolubles sont par exemple le rouge Soudan, le DC Red 17, le DC Green 6, le *-carotène, l'huile de soja, le brun Soudan, le DC Yellow 11, le DC Violet 2, le DC orange 5, le jaune quinoléine. Les colorants hydrosolubles sont par exemple le jus de betterave, le bleu de méthylène.

**ii) <u>Matériau à effet</u>**

La couche adhésive et la couche polymérique selon l'invention peuvent contenir au moins un matériau à effet optique spécifique. Cet effet est différent d'un simple effet de teinte conventionnel, c'est-à-dire unifié et stabilisé tel que

produit par les matières colorantes classiques décrite plus haut comme par exemple les pigments monochromatiques. Au sens de l'invention, « stabilisé » signifie dénué d'effet de variabilité de la couleur avec l'angle d'observation ou encore en réponse à un changement de température.

Ce matériau est présent en quantité suffisante pour produire un effet optique perceptible à l'oeil nu. Avantageusement, il s'agit d'un effet choisi parmi les effets goniochromatique, métallique et notamment miroir, soft-focus, arc en ciel et/ou thermochrome et/ou photochrome.

Par exemple, ce matériau peut être choisi parmi les particules à reflet métallique, les agents de coloration goniochromatiques, les pigments diffractants, les agents thermochromes, photochromiques les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Particules à reflet métallique

Par « particules à reflet métallique », on désigne des particules dont la nature, la taille, la structure et l'état de surface leur permettent de réfléchir la lumière incidente notamment de façon non iridescente.

Les particules présentant une surface extérieure sensiblement plane conviennent également, car elles peuvent plus facilement donner naissance, si leur taille, leur structure et leur état de surface le permettent, à une réflexion spéculaire intense que l'on peut alors qualifier d'effet miroir.

Les particules à reflet métallique utilisables dans l'invention, peuvent par exemple réfléchir la lumière dans toutes les composantes du visible sans absorber de manière significative une ou plusieurs longueurs d'ondes. La réflectance spectrale de ces particules peut par exemple être supérieure à 70 % dans l'intervalle 400-700 nm, et mieux d'au moins 80 %, voire 90 % ou encore 95 %.

Ces particules ont généralement une épaisseur inférieure ou égale à 1 μm, notamment inférieure ou égale à 0,7 μm, en particulier inférieure ou égale à 0,5 μm.

La proportion totale en particules à reflet métallique est notamment inférieure ou égale à 20 % en poids et en particulier inférieure ou égale à 10 % en poids par rapport au poids total de la première composition ou du film organique et/ou minéral.

Les particules à reflet métallique utilisables dans l'invention sont en particulier choisies parmi:

- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures.

Parmi les dérivés métalliques pouvant être présents dans lesdites particules, on peut citer notamment les oxydes métalliques tels que par exemple les oxydes de titane, notamment $TiO_2$, de fer notamment $Fe_2O_3$, d'étain, de chrome, le sulfate de baryum et les composés suivants : $MgF_2$, $CrF_3$, ZnS, ZnSe, $SiO_2$, $Al_2O_3$, MgO, $Y_2O_3$, $SeO_3$, SiO, $HfO_2$, $ZrO_2$, $CeO_2$, $Nb_2O_5$, $Ta_2O_5$, $MoS_2$ et leurs mélanges ou alliages.

Selon une première variante, les particules à reflet métallique peuvent être composées d'au moins un métal tel que défini précédemment, d'au moins un dérivé métallique tel que défini précédemment ou bien encore d'un de leurs mélanges.

Ces particules peuvent être au moins partiellement recouvertes par une couche d'un autre matériau, par un exemple de matériau transparent tel que notamment du colophane, de la silice, des stéarates, des polysiloxanes, des résines polyesters, des résines époxydiques, des résines polyuréthanes et des résines acryliques.

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Selon une seconde variante, ces particules peuvent être des particules comportant un substrat et qui présentent donc une structure multicouche par exemple bicouche. Ce substrat peut être organique ou minéral, naturel ou synthétique, monomatière ou multimatériaux, plein ou creux. Lorsque le substrat est synthétique, il peut être réalisé avec une forme favorisant la formation d'une surface réfléchissante après revêtement, notamment après le dépôt d'une couche de matériaux à reflet métallique. Le substrat peut, par exemple, présenter une surface plane et la couche de matériaux à reflet métallique une épaisseur sensiblement uniforme.

Le substrat peut être en particulier choisi parmi les métaux et les dérivés métalliques tels que cités précédemment, et également parmi les verres, les céramiques, les alumines, les silices, les silicates et notamment aluminosilicates et les borosilicates, le mica synthétique tel que le fluorophlogopite, et leurs mélanges, cette liste n'étant pas limitative. La couche à reflet métallique peut enrober en totalité ou en partie le substrat et cette couche peut être au moins partiellement recouverte par une couche d'un autre matériau, par exemple un matériau transparent notamment tel que cité précédemment. Selon un mode de réalisation particulier, cette couche à reflet métallique enrobe en totalité, directement ou indirectement, c'est-à-dire avec interposition d'au moins une couche intermédiaire métallique ou non, le substrat.

Les métaux ou dérivés métalliques pouvant être utilisés dans la couche réfléchissante sont tels que définis précédemment. Par exemple, elle peut être formée d'au moins un métal choisi parmi l'argent, l'aluminium, le chrome, le nickel, le molybdène, l'or, le cuivre, l'étain, le magnésium et leurs mélanges (alliages). On utilise plus particulièrement l'argent, le chrome, le nickel, le molybdène, et leurs mélanges.

A titre illustratif de ce second type de particules, on peut plus particulièrement citer :

Des particules de verre recouvertes d'une couche métallique notamment celles décrites dans les documents JP-A-09188830, JP-A-10158450, JP-A-10158541, JP-A-07258460 et JP-A-05017710.

A titre illustratif de ces particules comportant un substrat de verre, on peut citer celles revêtues respectivement d'argent, d'or ou de titane, en forme de plaquettes, commercialisées par la société NIPPON SHEET GLASS sous les dénominations MICROGLASS METASHINE. Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société. Celles revêtues soit d'oxyde de fer brun, soit d'oxyde de titane, d'oxyde d'étain ou d'un de leur mélange comme celles commercialisées sous la dénomination REFLECKS par la société ENGELHARD ou celles commercialisées sous la référence METASHINE MC 2080GP par la société NIPPON SHEET GLASS.

Ces particules de verre recouvertes de métaux peuvent être enrobées de silice comme celles commercialisées sous la dénomination METASHINE série PSS1 ou GPS1 par la société NIPPON SHEET GLASS.

Des particules à substrat de verre sphérique revêtu ou non par un métal, notamment celles vendues sous la dénomination PRIZMALITE MICROSPHERE par la société PRIZMALITE INDUSTRIES.

Conviennent également à l'invention, les pigments de la gamme METASHINE 1080R commercialisée par la société NIPPON SHEET GLASS CO. LTD.

Ces pigments, plus particulièrement décrits dans la demande de brevet JP 2001-11340, sont des paillettes de verre C-GLASS comprenant 65 à 72 % de $SiO_2$, recouvertes d'une couche d'oxyde de titane de type rutile ($TiO_2$). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de $TiO_2$.

Des particules comportant un substrat de borosilicate enrobé d'argent, encore appelées « nacres blanches ».

Des particules à substrat métallique tel que l'aluminium, le cuivre, le bronze, en forme de plaquettes, sont vendues sous la dénomination commerciale STARBRITE par la société SILBERLINE et sous la dénomination VISIONAIRE par la société ECKART. Des particules comportant un substrat de mica synthétique revêtu de dioxyde de titane, et par exemple les particules de dimension comprise entre 80 et 100 μm, comportant un substrat de mica synthétique (fluorophlogopite) revêtu de dioxyde de titane représentant 12 % du poids total de la particule, vendues sous la dénomination PROMINENCE par la société NIHON KOKEN.

Les particules à reflet métallique peuvent encore être choisies parmi les particules formées d'un empilement d'au moins deux couches à indices de réfraction différents. Ces couches peuvent être de nature polymérique ou métallique et notamment inclure au moins une couche polymérique.

Ainsi, les particules à effet métallique peuvent être des particules dérivant d'un film polymérique multicouche.

Le choix des matériaux destinés à constituer les différentes couches de la structure multicouche est bien entendu effectué de manière à conférer l'effet métallique souhaité aux particules ainsi formées.

De telles particules sont notamment décrites dans WO 99/36477, US 6,299,979 et US 6,387,498 et plus particulièrement identifiées ci-après dans le chapitre goniochromatique.

Pigments diffractants

Par « pigment diffractant », on désigne au sens de la présente invention un pigment capable de produire une variation de couleur selon l'angle d'observation lorsqu'éclairé par de la lumière blanche, en raison de la présence d'une structure qui diffracte la lumière.

Un pigment diffractant peut comporter un réseau de diffraction, capable par exemple de diffracter dans des directions définies un rayon de lumière monochromatique incident.

Le réseau de diffraction peut comporter un motif périodique, notamment une ligne, la distance entre deux motifs adjacents étant du même ordre de grandeur que la longueur d'onde de la lumière incidente.

Lorsque la lumière incidente est polychromatique, le réseau de diffraction va séparer les différentes composantes spectrales de la lumière et produire un effet arc-en-ciel.

On pourra utilement se reporter concernant la structure des pigments diffractants à l'article « Pigments Exhibiting Diffractive Effects » d'Alberto Argoitia and Matt Witzman, 2002, Society of Vacuum coaters, 45th Annual Technical Conference Proceedings 2002.

Le pigment diffractant peut être réalisé avec des motifs ayant différents profils, notamment triangulaires, symétriques ou non, en créneaux, de largeur constante ou non, sinusoïdaux.

La fréquence spatiale du réseau et la profondeur des motifs seront choisies en fonction du degré de séparation des différents ordres souhaités. La fréquence peut varier par exemple entre 500 et 3000 lignes par mm.

De préférence, les particules du pigment diffractant présentent chacune une forme aplatie, et notamment sont en forme de plaquette.

Une même particule de pigment peut comporter deux réseaux de diffraction croisés, perpendiculaires ou non.

Une structure possible pour le pigment diffractant peut comporter une couche d'un matériau réfléchissant, recouverte au moins d'un côté d'une couche d'un matériau diélectrique. Ce dernier peut conférer une meilleure rigidité et durabilité au pigment diffractant. Le matériau diélectrique peut alors être choisi par exemple parmi les matériaux suivants : $MgF_2$, $SiO_2$, $Al_2O_3$, $AlF_3$, $CeF_3$, $LaF_3$, $NdF_3$, $SmF_2$, $BaF_2$, $CaF_2$, LiF et leurs associations. Le matériau réfléchissant peut être choisi par exemple parmi les métaux et leurs alliages et aussi parmi les matériaux réfléchissants non métalliques. Parmi les métaux pouvant être utilisés, on peut citer Al, Ag, Cu, Au, Pt, Sn, Ti, Pd, Ni, Co, Rd, Nb, Cr et leurs composés, associations ou alliages. Un tel matériau réfléchissant peut, seul, constituer le pigment diffractant qui sera alors monocouche.

En variante, le pigment diffractant peut comporter une structure multicouche comportant un noyau d'un matériau diélectrique recouvert d'une couche réfléchissante au moins d'un côté, voire encapsulant complètement le noyau. Une couche d'un matériau diélectrique peut également recouvrir la ou les couches réfléchissantes. Le matériau diélectrique utilisé est alors de préférence inorganique, et peut être choisi par exemple parmi les fluorures métalliques, les oxydes métalliques, les sulfures métalliques, les nitrures métalliques, les carbures métalliques et leurs associations. Le matériau diélectrique peut être à l'état cristallin, semi-cristallin ou amorphe. Le matériau diélectrique, dans cette configuration, peut par exemple être choisi parmi les matériaux suivants : $MgF_2$, SiO, $SiO_2$, $Al_2O_3$, $TiO_2$, WO, AIN, BN, B4C, WC, TiC, TiN, $N_4Si_3$, ZnS, des particules de verre, des carbones de type diamant et leurs associations.

Le pigment diffractant utilisé peut notamment être choisi parmi ceux décrits dans la demande de brevet américain US 2003/0031870 publiée le 13 février 2003.

Un pigment diffractant peut comporter par exemple la structure suivante :

> $MgF_2$/Al/$MgF_2$, un pigment diffractant ayant cette structure étant commercialisé sous la dénomination SPECTRAFLAIR 1400 Pigment Silver par la société FLEX PRODUCTS, ou SPECTRAFLAIR 1400 Pigment Silver FG. La proportion en poids du $MgF_2$ peut être comprise entre 80 et 95 % du poids total du pigment.

Agents de coloration goniochromatiques

Au sens de l'invention, un agent de coloration goniochromatique permet d'observer un changement de couleur, encore appelé « color flop », en fonction de l'angle d'observation, supérieur à celui que l'on peut rencontrer avec des nacres. On peut utiliser simultanément un ou plusieurs agents de coloration goniochromatique.

L'agent de coloration goniochromatique peut être choisi de manière à présenter un changement de couleur relativement important avec l'angle d'observation.

L'agent de coloration goniochromatique peut ainsi être choisi de telle sorte que l'on puisse observer, pour une variation de l'angle d'observation comprise entre 0° et 80° sous un éclairage à 45°, une variation de couleur $\Delta E$ de la composition cosmétique, mesurée dans l'espace colorimétrique CIE 1976, d'au moins 2.

L'agent de coloration goniochromatique peut également être choisi de telle sorte que l'on puisse observer, pour un éclairage à 45° et une variation de l'angle d'observation comprise entre 0° et 80°, une variation Dh de l'angle de teinte de la composition cosmétique, dans le plan CIE 1976, d'au moins 30°, voire au moins 40° ou au moins 60°,

voire encore d'au moins 100°.

L'agent de coloration goniochromatique peut être choisi par exemple parmi les structures multicouches interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al_2O_3$, MgO, $Y_2O_3$, $S_2O_3$, SiO, $HfO_2$, $ZrO_2$, $CeO_2$, $Nb_2O_5$, $Ta_2O_5$, $TiO_2$, Ag, Al, Au, Cu, Rb, Ti, Ta, W, Zn, $MoS_2$, cryolithe, alliages, polymères et leurs associations.

La structure multicouche peut présenter ou non, par rapport à une couche centrale, une symétrie au niveau de la nature chimique des couches empilées.

Des exemples de structures multicouche interférentielles symétriques utilisables dans des compositions réalisées conformément à l'invention sont par exemple les structures suivantes : $Al/SiO_2/Al/SiO_2/Al$, des pigments ayant cette structure étant commercialisés par la société DUPONT DE NEMOURS ; $Cr/MgF_2/Al/MgF_2/Cr$, des pigments ayant cette structure étant commercialisés sous la dénomination CHROMAFLAIR par la société FLEX ; $MoS_2/SiO_2/Al/SiO_2/MoS^2$ ; $Fe_2O_3/SiO_2/Al/SiO_2/Fe_2O_3$, et $Fe_2O_3/SiO_2/Fe_2O_3/SiO_2/Fe_2O_3$, des pigments ayant ces structures étant commercialisés sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$ ; $TiO_2/SiO_2/TiO_2$ et $TiO_2/Al_2O_3/TiO_2$ ; $SnO/TiO_2/SiO_2/TiO_2/SnO$ ; $Fe_2O_3/SiO_2/Fe_2O_3$ ; SnO/mica/$TiO_2/SiO_2/TiO_2$/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure $Fe_2O_3/SiO_2/Al/\ SiO_2/Fe_2O_3$ on passe du doré-vert au gris-rouge pour des couches de $SiO_2$ de 320 à 350 nm ; du rouge au doré pour des couches de $SiO_2$ de 380 à 400 nm ; du violet au vert pour des couches de SiO2 de 410 à 420 nm ; du cuivre au rouge pour des couches de $SiO_2$ de 430 à 440 nm.

On peut encore utiliser des agents de coloration goniochromatique à structure multicouche comprenant une alternance de couches polymériques.

A titre illustratif, des matériaux pouvant constituer les différentes couches de la structure multicouche, on peut citer, cette liste n'étant pas limitative: le naphthalate de polyéthylène (PEN) et ses isomères par exemple 2,6-, 1,4-, 1,5-, 2,7- et 2,3-PEN, les téréphthalates de polyalkylene, des polyimides, des polyéthérimides, des polystyrènes atactiques, des polycarbonates, des polyméthacrylates et des polyacrylates d'alkyle, du polystyrène syndiotactique (sPS), des poly-alpha-méthylstyrène syndiotactiques, du polydichlorostyrène syndiotactique, copolymères et mélange de ses polystyrènes, des dérivés de cellulose, des polymères polyalkylènes, des polymères fluorés, des polymères chlorés, des polysulfones, des polyéthersulfones, des polyacrylonitriles, des polyamides, des résines siliconées, des résines époxy, de l'acétate de polyvinyle, des polyéthers-amides, des résines ionomériques, des élastomères et polyuréthanes. Conviennent également des copolymères, par exemple copolymères de PEN (par exemple, copolymères de 2,6-, 1,4-, 1,5-, 2,7-, et/ou 2,3-acide naphtalène dicarboxylique ou ses esters avec (a) acide téréphtalique ou ses esters ; (b) l'acide isophtalique ou ses esters ; (c) acide phtalique ou ses esters ; (d) des alcanes glycols ; (e) des cycloalkanes glycols (par exemple le cyclohexane diméthanol diol) ; (f) des acides alcanes dicarboxyliques ; et/ou (g) des acides cycloalkanes dicarboxylique, des copolymères de téréphthalates de polyalkylène et des copolymères de styrène. En outre, chaque couche individuelle peut inclure des mélanges de deux ou plusieurs polymères ou copolymères précédents. Le choix des matériaux destinés à constituer les différentes couches de la structure multicouche est bien entendu effectué de manière à conférer l'effet optique souhaité aux particules ainsi formées.

On peut citer, à titre d'exemple de pigments à structure multicouche polymérique, ceux commercialisés par la société 3M sous la dénomination COLOR GLITTER.

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celle commercialisées sous la dénomination HELICONE® HC par la société WACKER.

Ces agents peuvent également être sous forme de fibres goniochromatiques dispersées. De telles fibres peuvent par exemple présenter une taille comprise entre 50 μm et 700 μm, par exemple d'environ 300 μm. En particulier, on peut utiliser des fibres interférentielles à structure multicouche. Des fibres à structure multicouche de polymères sont notamment décrites dans les documents EP-A-0 921 217, EP-A-0 686 858 et US-A-5,472,798. La structure multicouche peut comporter au moins deux couches, chaque couche, indépendamment ou non de la (ou les) autre(s) couche(s), étant réalisée en au moins un polymère de synthèse. Les polymères présents dans les fibres peuvent avoir un indice de réfraction allant de 1,30 à 1,82 et mieux allant de 1,35 à 1,75. Les polymères préférés pour constituer les fibres sont

les polyesters tels que le polyéthylène téréphtalate, le polyéthylène naphtalate, le polycarbonate ; les polymères acryliques comme le polyméthacrylate de méthyle ; les polyamides.

Des fibres goniochromatiques à structure bicouche polyéthylène téréphtalate/nylon-6 sont commercialisées par la société TEIJIN sous la dénomination MORPHOTEX.

Dans une variante, cet agent de coloration goniochromatique peut être associé à au moins un pigment diffractant.

La combinaison de ces deux matériaux résulte en une composition ou un film qui présente une variabilité de la couleur accrue, donc qui est susceptible de permettre à un observateur de percevoir un changement de couleur, voire un mouvement de couleur, dans de nombreuses conditions d'observation et d'illumination.

Le rapport pondéral du pigment diffractant par rapport à l'agent de coloration goniochromatique est de préférence compris entre 85/15 et 15/85, mieux entre 80/20 et 20/80, mieux encore 60/40 et 40/60, par exemple de l'ordre de 50/50. Un tel rapport est favorable à l'obtention d'un effet arc-en-ciel et d'un effet goniochromatique soutenus.

Azurants optiques

Les azurants optiques sont des composés bien connus de l'homme du métier.

De tels composés sont notamment décrits dans « Fluorescent Whitening Agent, Encyclopedia of Chemical Technology, Kirk-Othmer », vol. 11, p. 227-241, 4ème édition, 1994, Wiley. On peut plus particulièrement les définir comme des composés qui absorbent essentiellement dans l'UVA entre 300 et 390 nm et réémettent essentiellement entre 400 et 525 nm. Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et leurs mélanges. De tels composés sont facilement disponibles commercialement.

On peut citer par exemple : le dérivé stilbénique de naphto-triazole vendu sous la dénomination commerciale « Tinopal GS », le di-styryl-4,4' biphényle sulfonate di-sodique (nom CTFA : disodium distyrylbiphenyl disulfonate) vendu sous la dénomination commerciale « Tinopal CBS-X », le dérivé cationique d'aminocoumarine vendu sous la dénomination commerciale « Tinopal SWN CONC. », le 4,4'-bis[(4,6-dianilino-1,3,5-triazin-2-yl)amino]stilbène-2,2'-disulfonate de sodium vendu sous la dénomination commerciale « Tinopal SOP », le 4,4'-bis-[(4-anilino-6-bis(2-dydroxyéthyl)amino-1,3,5-triazin-2-yl)amino]stilbène-2,2'-disulfonic acide vendu sous la dénomination commerciale « Tinopal UNPA-GX », le 4,4'-bis-[anilino-6-morpholine-1,3,5-triazin-2-yl)amino]stilbène vendu sous la dénomination commerciale « Tinopal AMS-GX », le 4,4'-bis-[(4-anilino-6-(2-hydroxyéthyl)méthylamino-1,3,5-triazin-2-yl)amino]stilbène-2,2'-disodium sulfonate vendu sous la dénomination commerciale « Tinopal 5BM-GX », tous par la société CIBA Spécialités Chimiques, le 2,5 thiophène di-yl bis(5 ter-butyl-1,3 benzoxazole) vendu sous la dénomination commerciale « Uvitex OB » par la société CIBA, le dérivé anionique du di-aminostilbène en dispersion dans l'eau vendu sous la dénomination commerciale « Leucophor BSB liquide » par la société CLARIANT, les laques d'azurant optique vendues sous la dénomination commerciale « COVAZUR » par la société WACKHERR.

Les azurants optiques utilisables dans la présente invention peuvent aussi se présenter sous la forme de copolymères, par exemple d'acrylates et/ou de méthacrylates, greffés par des groupements azurants optiques comme décrits dans la demande FR 9 910 942.

Ils peuvent être utilisés tels quels ou introduits dans le film sous forme de particules et/ou de fibres recouvertes dudit azurant, telles que celles décrites ci-après.
En particulier, on peut utiliser les fibres recouvertes d'azurant optique telles que commercialisées par la société LCW sous la référence commerciale Fiberlon 54 $ZO_3$, ayant une longueur d'environ 0,4 mm et un titre de 0,5 deniers.

Matériau à effet relief

L'effet relief peut ou non être associé à un effet optique. Un matériau de ce type est généralement présent en quantité suffisante pour conférer un effet relief perceptible au toucher voire à l'oeil nu. Il peut notamment s'agir d'un effet rugueux et/ou martelé.

Matériau conférant un aspect rugueux

Les particules de forme sensiblement sphérique ou ovoïdale peuvent conférer un toucher doux au maquillage. Avantageusement, les particules solides ont une forme sensiblement sphérique, pour permettre leur bonne répartition lors de leur application. Les particules solides utilisées selon l'invention peuvent avoir une taille moyenne allant de 2,5 $\mu$m à 5 mm, et mieux de 50 $\mu$m à 2 mm. Plus les particules sont de petites tailles, plus la tenue des particules est satisfaisante. L'emploi de particules est également compatible avec la réalisation de motifs.

Les particules solides peuvent être en tout matériau satisfaisant les propriétés de densités définies précédemment. Par exemple, les particules solides peuvent être en un matériau choisi parmi le verre, l'oxyde de zirconium, le carbure de tungstène, les plastiques comme les polyuréthanes, les polyamides, le polytétrafluoroéthylène, le polypropylène, les métaux comme l'acier, le cuivre, le laiton, le chrome, le marbre, l'onyx, le jade, la nacre naturelle, les pierres précieuses (diamant, émeraude, rubis, saphir), l'améthyste, l'aigue-marine. On utilise de préférence des billes de verres telles que celles vendues sous la dénomination « SILIBEADS® » par la société SIGMUND LINDNER ; ces billes ont en outre pour avantage de conférer également un effet brillant et un scintillement au maquillage.

Les particules solides, déformables ou non, peuvent être pleines ou creuses, incolores ou colorées, enrobées ou non.

En ce qui concerne les fibres utilisables selon l'invention, il peut s'agir de fibres d'origine synthétique ou naturelle, minérale ou organique.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Il peut notamment s'agir de fibres utilisées dans la fabrication des textiles et notamment des fibres de soie, de coton, de laine, de lin, des fibres de cellulose, notamment extraite du bois, des légumes ou des algues, de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

Matériau conférant un aspect martelé

Les inventeurs ont également constaté qu'il était possible de conditionner dans l'invention un matériau comprenant un mélange silice pyrogéné, pigment métallique et composé organopolysiloxane pour lui conférer un aspect martelé.

Un tel mélange est notamment décrit dans la demande de brevet EP 1 040 813.

**iii) Matériau à effet olfactif**

Avantageusement, l'article selon l'invention peut également être doté de propriétés olfactives par incorporation notamment dans l'une au moins des couches d'au moins un matériau odorant ou encore substance parfumante.

La substance parfumante peut être choisie parmi toute substance odoriférante bien connue de l'homme du métier, et notamment parmi les huiles essentielles et/ou les essences.

Ce matériau olfactif peut, si nécessaire, être introduit *via* un solvant-plastifiant.

On entend par « solvant-plastifiant » un composé qui solubilise au moins partiellement le matériau olfactif et qui est susceptible de s'évaporer lentement.

Le solvant-plastifiant peut être choisi parmi des glycols tels que le dipropylène glycol, l'éthyldiglycol, le n-propylglycol, le n-butylglycol, le méthyldiglycol, le n-butyldiglycol, des alcools tels que le cyclohexanol, l'éthyl-2 butanol, le méthoxy-3 butanol, l'éthyl-2 hexanol, le phénoxyéthanol, des esters, tels que le monoacétate de glycol, l'acétate d'éthylglycol, l'acétate de n-butylglycol, l'acétate d'éthyldiglycol, l'acétate de n-butyldiglycol, l'abiétate de méthyle, le myristate d'isopropyle, le diacétate de propylène glycol, l'acétate d'éther méthylique du propylène glycol, des éthers de glycols tels que l'éther méthylique du dipropylène glycol, l'éther butylique du dipropylène glycol, seuls ou en mélange.

L'une quelconque des couches de l'article selon l'invention peut également contenir un ou plusieurs additifs de formulation couramment utilisés en cosmétique et plus spécialement dans le domaine cosmétique et/ou soin des ongles. Ils peuvent notamment être choisis parmi les vitamines, les oligo-éléments, les adoucissants, les séquestrants, les agents alcalinisants ou acidifiants, les agents mouillants, les agents épaississants, les agents dispersants, les anti-mousses, les agents d'étalement, les co-résines, les conservateurs, les filtres UV, les actifs, les agents hydratants, les neutralisants, les stabilisants, les antioxydants et leurs mélanges.

Ainsi, ils peuvent notamment incorporer, à titre d'actifs, des agents durcissants ou renforçateur pour matières kératiniques, des actifs favorisant la pousse de l'ongle tel que le méthylsulfonylméthane et/ou des actifs pour traiter des affections diverses localisées au niveau de l'ongle, comme par exemple des actifs antimycotiques ou antimicrobiens.

Les quantités de ces différents ingrédients sont celles classiquement utilisées dans ce domaine et par exemple de 0,01 à 20 % en poids, et notamment de 0,01 à 10 % en poids par rapport au poids total de la feuille souple et/ou du matériau adhésif.

L'invention est illustrée en détail dans l'exemple suivant qui est présenté à titre illustratif et non limitatif de

l'invention.

Sauf indication contraire, les quantités sont données en pourcentage en poids en matière première par rapport au poids total de la composition.

L'invention est illustrée plus en détail dans les exemples suivants qui sont présentés à titre illustratif et non limitatif de l'invention.

Sauf indication contraire, les quantités sont données en pourcentage en poids par rapport au poids total de la composition.

**Exemple 1**

| | |
|---|---|
| Acétobutyrate de cellulose (1) | 10,0 % |
| Gomme benjoin (2) | 15,0 % |
| Ethanol (3) | 37,5 % |
| Acétate d'éthyle (4) | 37,5 % |

(1) CAB 381 0,5 commercialisée par la société Eastman Chemical
(2) commercialisée par la société WoodFininshing Enterprises
(3) commercialisé par la société Tereos
(4) commercialisé par la société Rhodia

**Exemple 2**

| | |
|---|---|
| Acétobutyrate de cellulose (1) | 4,2 % |
| Ethanol (3) | 46,2 % |
| Acétate d'éthyle (4) | 25,8 % |
| Résine Sandaraque (nord africaine) (5) | 19,8 % |
| Triglycérides d'acides caprylique/caprique (6) | 4,0 % |

(5) commercialisée par la société WoodFimshing Enterprises
(6) commercialisés par la société Cognis sous la référence Myritol 318

**Exemple 3**

| | |
|---|---|
| Acétobutyrate de cellulose (1) | 7,5 % |
| Gomme Benjoin (2) | 10,0 % |
| Ethanol (3) | 25,0 % |
| Acétate d'éthyle (4) | 47,5 % |
| Résine Sandaraque (nord africaine) (5) | 10,0 % |

**Exemple 4**

| | |
|---|---|
| Ethanol (3) | 19,5 % |
| Acétate d'éthyle (4) | 56,0 % |
| Ethylguar (6) | 6,5 % |
| Gomme élémi distillée purifiée concassée (7) | 15,5 % |
| Oxyde de fer rouge (8) | 2,5 % |
| (6) N-Hance AG 200 commercialisée par la société Hercules<br>(7) commercialisée par la société Emiga<br>(8) commercialisé par la société LCW sous la référence Natpure Red | |

**Exemple 5**

| | |
|---|---|
| Triglycérides d'acides caprylique/caprique (6) | 4,0 % |
| Ethanol d'origine végétale (3) | 45,7 % |
| Acétate d'éthyle d'origine végétale (4) | 25,3 % |
| Acétobutyrate de cellulose (1) | 4,2 % |
| Résine Sandaraque Nord Africaine (5) | 19,8 % |
| Pigments | 1,0 % |

Pour obtenir les compositions des exemples, les ingrédients sont mélangés puis placés sous agitation jusqu'à solubilisation de l'ensemble des composés (hors pigments), et jusqu'à obtenir une bonne dispersion des pigments (pour les formules comportant des pigments).

Après séchage, les compositions des exemples 1 à 5 forment des films durs, brillants et adhérents Les composés dans le cadre de l'invention ne s'avèrent pas préjudiciable aux qualités annexes attendues pour une composition cosmétique.

**Exemple 6**

Dans cet exemple, un premier film polymérique est réalisé à partir d'un latex d'Hévéa déprotéinisé (9) d'extrait sec d'environ 60 %. L'enduction du latex d'Hévéa est réalisée à 150 $\mu$m humide sur carte de contraste, commercialisée par la société BYK Gardner sous le nom du produit Prüfkarte 2853.

Une seconde composition (voir tableau suivant) est enduite à 300 $\mu$m humide sur un contre adhésif (Scotchpak 1022 Release liner 3.0 mil disponible chez 3M).

| | |
|---|---|
| Ethylguar (6) | 5 % |
| Gomme Elemi (7) | 15 % |
| Triglycérides d'acides caprylique/caprique (6) | 10 % |
| Acétate d'éthyle (4) | 70 % |
| (9) commercialisée par la société SRI Hybrid | |

Après séchage des deux films, le film de latex est mis en contact avec le film de la composition du tableau. Une pression est exercée pour faire adhérer les films entre eux. Ensuite, on retire avec précaution la carte de contraste

puis le contre-adhésif. On obtient un laminé adhésif souple et résistant, permettant au laminé adhésif d'être étiré facilement lors de l'application sur les ongles.

## Revendications

1. Vernis à ongles comprenant un milieu solvant constitué d'un ou plusieurs solvants d'origine végétale, au moins un alkyléther ou un ester de polysaccharide et au moins une résine végétale, **caractérisé en ce que** le milieu solvant est choisi parmi un mélange d'éthanol et d'acétate d'éthyle.

2. Vernis à ongles comprenant un milieu solvant constitué d'un ou plusieurs solvants d'origine végétale, au moins un alkyléther ou un ester de polysaccharide et au moins une résine végétale, **caractérisé en ce que** la résine végétale est choisie parmi la colophane et ses dérivés, les résines terpéniques, la gomme sandaraque, les dammars, l'élémi, les copals, le benjoin, la gomme mastic et leurs mélanges.

3. Vernis à ongles selon la revendication 2, **caractérisé en ce que** le milieu solvant d'origine végétale est choisi parmi les alcools, notamment l'éthanol et/ou les acétates, notamment l'acétate d'éthyle et/ou les lactates, notamment le lactate d'éthyle.

4. Vernis à ongles selon la revendication 2 ou 3, **caractérisé en ce que** le milieu solvant est choisi parmi un mélange d'éthanol et d'acétate d'éthyle.

5. Vernis à ongles selon l'une quelconque des revendications précédentes, **caractérisé en ce qu'**il comprend moins de 5 % d'eau en poids, voire moins de 3 %, voire moins de 1 % par rapport au poids total du vernis à ongles, voire en est complètement dépourvu.

6. Vernis à ongles selon l'une quelconque des revendications précédentes, **caractérisé en ce que** le milieu solvant est présent en une teneur comprise entre 1 et 99 % en poids, notamment entre 40 et 90 % en poids, en particulier entre 60 et 80 % en poids par rapport au poids total du vernis à ongles.

7. Vernis à ongles selon l'une quelconque des revendications précédentes, **caractérisé en ce que** l'alkyléther ou l'ester de polysaccharide est choisi parmi les acétobutyrates de cellulose, les acétopropionates de cellulose, les éthylcelluloses et les éthylguars.

8. Vernis à ongles selon l'une quelconque des revendications précédentes, **caractérisé en ce que** l'alkyléther ou l'ester de polysaccharide est présent en une teneur comprise entre 1 et 60 % en poids, notamment entre 2 et 30 % en poids, en particulier entre 4 et 10 % en poids par rapport au poids total du vernis à ongles.

9. Vernis à ongles selon la revendication 1 et 5 à 8 , **caractérisé en ce que** la résine végétale est choisie parmi la colophane et ses dérivés, les résines terpéniques, la gomme sandaraque, les dammars, l'élémi, les copals, le benjoin, la gomme mastic et leurs mélanges.

10. Vernis à ongles selon l'une quelconque des revendications précédentes, **caractérisé en ce que** la résine végétale est présente en une teneur comprise entre 1 et 60 % en poids, notamment entre 10 et 50 % en poids, en particulier entre 15 et 35 % en poids par rapport au poids total du vernis à ongles.

11. Vernis à ongles selon l'une quelconque des revendications précédentes, **caractérisé en ce qu'**il comprend en outre un ester d'acide et/ou d'alcool caprylique/caprique, par exemple le triglycéride d'acide caprylique et/ou caprique.

12. Vernis à ongles selon la revendication 11, **caractérisé en ce que** l'ester d'acide et/ou d'alcool caprylique/caprique est présent en une teneur comprise entre 0,5 à 15 % en poids, notamment de 1 à 10 % en poids, en particulier de 2 à 8 % en poids par rapport au poids total de la composition.

13. Vernis à ongles selon l'une quelconque des revendications précédentes, **caractérisé en ce qu'**il comprend moins de 5 % en poids de matière sèche de nitrocellulose par rapport au poids total du vernis à ongles, de préférence moins de 3 % en poids, de préférence moins de 1,5 % en poids, de préférence moins de 1 % en poids, voire en est totalement dépourvu.

**14.** Vernis à ongles selon l'une quelconque des revendications précédentes, **caractérisé en ce qu'**il comprend au moins une matière colorante, et en particulier en une teneur allant de 0,01 % à 50 % en poids, par rapport au poids du vernis à ongles, de préférence de 0,01 % à 30 % en poids.

**15.** Procédé cosmétique de maquillage ou de soin non thérapeutique des ongles comprenant l'application sur les ongles d'au moins une couche d'un vernis à ongles selon l'une quelconques des revendications précédentes.

**16.** Article souple comprenant en particulier dans une couche adhésive, au moins une résine végétale, au moins un plastifiant et de préférence au moins un solvant d'origine végétale, **caractérisé en ce que** le plastifiant est choisi parmi les esters d'acide caprylique et/ou caprique ou ester d'alcool caprylique et/ou caprique et notamment les triglycérides d'acide caprylique et/ou caprique.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006056558 A
- EP 0676451 A
- WO 2007080172 A
- US 5066484 A
- US 5145670 A
- JP 2005263656 A
- JP 2004315479 A
- FR 0302809
- EP 0898960 A
- US 6136296 A
- US 5929173 A
- EP 0542669 A
- EP 0787730 A
- EP 0787731 A
- WO 9608537 A
- JP 09188830 A
- JP 10158450 A
- JP 10158541 A
- JP 07258460 A
- JP 05017710 A
- JP 2001011340 A
- WO 9936477 A
- US 6299979 B
- US 6387498 B
- US 20030031870 A
- EP 0921217 A
- EP 0686858 A
- US 5472798 A
- FR 9910942
- EP 1040813 A


### Littérature non-brevet citée dans la description


- **Kirk-Othmer.** l'Encyclopedia of Chemical Technology. vol. 2, 53-63
- **D. Satas.** Handbook of Pressure Sensitive Adhesive Technology. 155-157
- **D. Satas.** Handbook of Pressure Sensitive Adhesive Technology
- **Alberto Argoitia ; Matt Witzman.** Pigments Exhibiting Diffractive Effects. *Society of Vacuum coaters, 45th Annual Technical Conference Proceedings 2002,* 2002
- Fluorescent Whitening Agent. **Kirk-Othmer.** Encyclopedia of Chemical Technology. Wiley, 1994, vol. 11, 227-241